# EUROPEAN PATENT SPECIFICATION

(11) **EP 2 370 109 B1**
(45) Date of publication and mention of the grant of the patent: **26.02.2014**
(21) Application number: 09831355.4
(22) Date of filing: 10.12.2009
(51) Int. Cl.: A61K 31/7088, A61K 38/17, A61P 35/00, C12Q 1/68, G01N 33/574, G01N 33/68, C12N 15/113

(54) **PROLIFERATION-ASSOCIATED MODULATION OF THE SPLICING OF THE INTEGRIN ALPHA 6 ISOFORMS**
PROLIFERATIONSASSOZIIERTE MODULATION DES SPLEISSENS VON INTEGRIN-ALPHA-6-ISOFORMEN
MODULATION ASSOCIÉE À LA PROLIFÉRATION D'ÉPISSAGE DES ISOFORMES INTÉGRINE ALPHA 6

(30) Priority: 10.12.2008 US 121337 P; 12.05.2009 US 177398 P
(43) Date of publication of application: 05.10.2011
(73) Proprietor: Socpra Sciences Santé Et Humaines S.E.C., Sherbrooke, Québec J1L 1E2 (CA)
(72) Inventor: BEAULIEU, Jean-François, Sherbrooke Québec J1E 2H1 (CA); HERRING, Elizabeth, North Hatley Québec J0B 2C0 (CA); BASORA, Nuria, Sherbrooke Québec J1K 1W5 (CA); GROULX, Jean-François, St-Nicolas Québec G7A 4Z6 (CA)
(74) Representative: Delorme, Nicolas
(86) International application number: PCT/CA2009/001802
(87) International publication number: WO 2010/066046

(56) References cited:
- DYDENSBORG ANDERS BONDO ET AL: "Integrin Î+-6BÎ4 inhibits colon cancer cell proliferation and c-Myc activity", BMC CANCER, BIOMED CENTRAL, LONDON, GB, vol. 9, no. 1, 9 July 2009 (2009-07-09), page 223, XP021057601, ISSN: 1471-2407, DOI: 10.1186/1471-2407-9-223
- SEGAT, D. ET AL.: 'Integrins alpha6Abeta1 and alpha6Bbeta1Promote Different Stages of Chondrogenic Differentiation.' J. BIOL. CHEM. vol. 277, no. 35, 30 August 2002, pages 31612 - 31622, XP008154762
- DELWEL, G. O. ET AL.: 'Expression and Function of the Cytoplasmic Variants of the Integrin alpha6 Subunit in Transfected K562 Cells.' J. BIOL. CHEM. vol. 268, no. 34, 05 December 1993, pages 25865 - 25875, XP008154764
- DYDENSBORG, A. B. ET AL.: 'CANADIAN DIGESTIVE DISEASES WEEK (CDDW) February 24-27th, 2006', 2006, BANFF, ALBERTA, CANADA. article 'The integrin alpha6 subunit is upregulated in adenocarcinomas of the human colon and undergoes alternative splicing reminiscent of that found in the crypt of the normal intestine.', XP008154778
- SASTRY, S. K. ET AL.: 'Integrin a Subunit Ratios, Cytoplasmic Domains, and Growth Factor Synergy Regulate Muscle Proliferation and Differentiation.' J. CELL BIOL. vol. 133, no. 1, 01 April 1996, pages 169 - 184, XP008154760
- DYDENSBORG, A. B. ET AL.: 'Integrin alpha6Bbeta4 inhibits colon cancer cell proliferation and c-Myc activity.' BMC CANCER vol. 9, 09 July 2009, page 223, XP021057601
- DYDENSBORG, A. B. ET AL.: 'Differential expression of the integrins alpha6Abeta4 and alpha6Bbeta4 along the crypt-villus axis in the human small intestine.' HISTOCHEM. CELL BIOL. vol. 131, 24 December 2008, pages 531 - 536, XP019714783

### SEQUENCE LISTING

<110> SOCPRA SCIENCES SANTE ET HUMAINES, S.E.C.
   BEAULIEU, Jean-Francois
   HERRING, Elizabeth
   BASORA, Nuria
   GROULX, Jean-François
<120> PROLIFERATION-ASSOCIATED MODULATION OF THE SPLICING OF THE INTERGRIN ALPHA 6 ISOFORMS
<130> 05006078-41PCT
<150> 61/121,337
   <151> 2008-12-10
<150> 61/177,398
   <151> 2009-05-12
<160> 13
<170> PatentIn version 3.5
<210> 1
   <211> 5810
   <212> DNA
   <213> Homo sapiens
<400> 1 SEQUENCE LISTING
<110> SOCPRA SCIENCES SANTE ET HUMAINES, S.E.C.

   BEAULIEU, Jean-François
   HERRING, Elizabeth
   BASORA, Nuria
   GROULX, Jean-François
<120> PROLIFERATION-ASSOCIATED MODULATION OF THE SPLICING OF THE INTERGRIN ALPHA 6 ISOFORMS
<130> 05006078-41PCT
<150> 61/121,337
   <151> 2008-12-10
<150> 61/177,398
   <151> 2009-05-12
<160> 13
<170> PatentIn version 3.5
<210> 1
   <211> 5810
   <212> DNA
   <213> Homo sapiens
<400> 1
   aacgggctca ttcagcggtc gcgagctgcc cgcgaggggg agcggccgga cggagagcgc      60
   gacccgtccc gggggtgggg ccgggcgcag cggcgagagg aggcgaaggt ggctgcggta     120
   gcagcagcgc ggcagcctcg gacccagccc ggagcgcagg gcggccgctg caggtccccg     180
   ctcccctccc cgtgcgtccg cccatggccg ccgccgggca gctgtgcttg ctctacctgt     240
   cggcggggct cctgtcccgg ctcggcgcag ccttcaactt ggacactcgg gaggacaacg     300
   tgatccggaa atatggagac cccgggagcc tcttcggctt ctcgctggcc atgcactggc     360
   aactgcagcc cgaggacaag cggctgttgc tcgtgggggc cccgcgggca gaagcgcttc     420
   cactgcagag agccaacaga acgggagggc tgtacagctg cgacatcacc gcccgggggc     480
   catgcacgcg gatcgagttt gataacgatg ctgaccccac gtcagaaagc aaggaagatc     540
   agtggatggg ggtcaccgtc cagagccaag gtccaggggg caaggtcgtg acatgtgctc     600
   accgatatga aaaaaggcag catgttaata cgaagcagga atcccgagac atctttgggc     660
   ggtgttatgt cctgagtcag aatctcagga ttgaagacga tatggatggg ggagattgga     720
   gcttttgtga tgggcgattg agaggccatg agaaatttgg ctcttgccag caaggtgtag     780
   cagctacttt tactaaagac tttcattaca ttgtatttgg agccccgggt acttataact     840
   ggaaagggat tgttcgtgta gagcaaaaga ataacacttt ttttgacatg aacatctttg     900
   aagatgggcc ttatgaagtt ggtggagaga ctgagcatga tgaaagtctc gttcctgttc     960
   ctgctaacag ttacttaggt ttttctttgg actcagggaa aggtattgtt tctaaagatg 1020
   agatcacttt tgtatctggt gctcccagag ccaatcacag tggagccgtg gttttgctga 1080
   agagagacat gaagtctgca catctcctcc ctgagcacat attcgatgga gaaggtctgg 1140
   cctcttcatt tggctatgat gtggcggtgg tggacctcaa caaggatggg tggcaagata 1200
   tagttattgg agccccacag tattttgata gagatggaga agttggaggt gcagtgtatg 1260
   tctacatgaa ccagcaaggc agatggaata atgtgaagcc aattcgtctt aatggaacca 1320
   aagattctat gtttggcatt gcagtaaaaa atattggaga tattaatcaa gatggctacc 1380
   cagatattgc agttggagct ccgtatgatg acttgggaaa ggtttttatc tatcatggat 1440
   ctgcaaatgg aataaatacc aaaccaacac aggttctcaa gggtatatca ccttattttg 1500
   gatattcaat tgctggaaac atggaccttg atcgaaattc ctaccctgat gttgctgttg 1560
   gttccctctc agattcagta actattttca gatcccggcc tgtgattaat attcagaaaa 1620
   ccatcacagt aactcctaac agaattgacc tccgccagaa aacagcgtgt ggggcgccta 1680
   gtgggatatg cctccaggtt aaatcctgtt ttgaatatac tgctaacccc gctggttata 1740
   atccttcaat atcaattgtg ggcacacttg aagctgaaaa agaaagaaga aaatctgggc 1800
   tatcctcaag agttcagttt cgaaaccaag gttctgagcc caaatatact caagaactaa 1860
   ctctgaagag gcagaaacag aaagtgtgca tggaggaaac cctgtggcta caggataata 1920
   tcagagataa actgcgtccc attcccataa ctgcctcagt ggagatccaa gagccaagct 1980
   ctcgtaggcg agtgaattca cttccagaag ttcttccaat tctgaattca gatgaaccca 2040
   agacagctca tattgatgtt cacttcttaa aagagggatg tggagacgac aatgtatgta 2100
   acagcaacct taaactagaa tataaatttt gcacccgaga aggaaatcaa gacaaatttt 2160
   cttatttacc aattcaaaaa ggtgtaccag aactagttct aaaagatcag aaggatattg 2220
   ctttagaaat aacagtgaca aacagccctt ccaacccaag gaatcccaca aaagatggcg 2280
   atgacgccca tgaggctaaa ctgattgcaa cgtttccaga cactttaacc tattctgcat 2340
   atagagaact gagggctttc cctgagaaac agttgagttg tgttgccaac cagaatggct 2400
   cgcaagctga ctgtgagctc ggaaatcctt ttaaaagaaa ttcaaatgtc actttttatt 2460
   tggttttaag tacaactgaa gtcacctttg acaccccaga tctggatatt aatctgaagt 2520
   tagaaacaac aagcaatcaa gataatttgg ctccaattac agctaaagca aaagtggtta 2580
   ttgaactgct tttatcggtc tcgggagttg ctaaaccttc ccaggtgtat tttggaggta 2640
   cagttgttgg cgagcaagct atgaaatctg aagatgaagt gggaagttta atagagtatg 2700
   aattcagggt aataaactta ggtaaacctc ttacaaacct cggcacagca accttgaaca 2760
   ttcagtggcc aaaagaaatt agcaatggga aatggttgct ttatttggtg aaagtagaat 2820
   ccaaaggatt ggaaaaggta acttgtgagc cacaaaagga gataaactcc ctgaacctaa 2880
   cggagtctca caactcaaga aagaaacggg aaattactga aaaacagata gatgataaca 2940
   gaaaattttc tttatttgct gaaagaaaat accagactct taactgtagc gtgaacgtga 3000
   actgtgtgaa catcagatgc ccgctgcggg ggctggacag caaggcgtct cttattttgc 3060
   gctcgaggtt atggaacagc acatttctag aggaatattc caaactgaac tacttggaca 3120
   ttctcatgcg agccttcatt gatgtgactg ctgctgccga aaatatcagg ctgccaaatg 3180
   caggcactca ggttcgagtg actgtgtttc cctcaaagac tgtagctcag tattcgggag 3240
   taccttggtg gatcatccta gtggctattc tcgctgggat cttgatgctt gctttattag 3300
   tgtttatact atggaagtgt ggtttcttca agagaaataa gaaagatcat tatgatgcca 3360
   catatcacaa ggctgagatc catgctcagc catctgataa agagaggctt acttctgatg 3420
   catagtattg atctacttct gtaattgtgt ggattcttta aacgctctag gtacgatgac 3480
   agtgttcccc gataccatgc tgtaaggatc cggaaagaag agcgagagat caaagatgaa 3540
   aagtatattg ataaccttga aaaaaaacag tggatcacaa agtggaacga aaatgaaagc 3600
   tactcatagc gggggcctaa aaaaaaaaag cttcacagta cccaaactgc tttttccaac 3660
   tcagaaattc aatttggatt taaaagcctg ctcaatccct gaggactgat ttcagagtga 3720
   ctacacacag tacgaaccta cagttttaac tgtggatatt gttacgtagc ctaaggctcc 3780
   tgttttgcac agccaaattt aaaactgttg gaatggattt ttctttaact gccgtaattt 3840
   aactttctgg gttgccttta tttttggcgt ggctgactta catcatgtgt tggggaaggg 3900
   cctgcccagt tgcactcagg tgacatcctc cagatagtgt agctgaggag gcacctacac 3960
   tcacctgcac taacagagtg gccgtcctaa cctcgggcct gctgcgcaga cgtccatcac 4020
   gttagctgtc ccacatcaca agactatgcc attggggtag ttgtgtttca acggaaagtg 4080
   ctgtcttaaa ctaaatgtgc aatagaaggt gatgttgcca tcctaccgtc ttttcctgtt 4140
   tcctagctgt gtgaatacct gctcacgtca aatgcataca agtttcattc tccctttcac 4200
   taaaacacac aggtgcaaca gacttgaatg ctagttatac ttatttgtat atggtattta 4260
   ttttttcttt tctttacaaa ccattttgtt attgactaac aggccaaaga gtctccagtt 4320
   tacccttcag gttggtttaa tcaatcagaa ttagagcatg ggaggtcatc actttgacct 4380
   aaattattta ctgcaaaaag aaaatcttta taaatgtacc agagagagtt gttttaataa 4440
   cttatctata aactataacc tctccttcat gacagcctcc accccacaac ccaaaaggtt 4500
   taagaaatag aattataact gtaaagatgt ttatttcagg cattggatat tttttacttt 4560
   agaagcctgc ataatgtttc tggatttcat actgtaacat tcaggaattc ttggagaaaa 4620
   tgggtttatt cactgaactc tagtgcggtt tactcactgc tgcaaatact gtatattcag 4680
   gacttgaaag aaatggtgaa tgcctatggt ggatccaaac tgatccagta taagactact 4740
   gaatctgcta ccaaaacagt taatcagtga gtcgatgttc tattttttgt tttgtttcct 4800
   cccctatctg tattcccaaa aattactttg gggctaattt aacaagaact ttaaattgtg 4860
   ttttaattgt aaaaatggca gggggtggaa ttattactct atacattcaa cagagactga 4920
   atagatatga aagctgattt tttttaatta ccatgcttca caatgttaag ttatatgggg 4980
   agcaacagca aacaggtgct aatttgtttt ggatatagta taagcagtgt ctgtgttttg 5040
   aaagaataga acacagtttg tagtgccact gttgttttgg gggggctttt ttcttttcgg 5100
   aaatcttaaa ccttaagata ctaaggacgt tgttttggtt gtactttgga attcttagtc 5160
   acaaaatata ttttgtttac aaaaatttct gtaaaacagg ttataacagt gtttaaagtc 5220
   tcagtttctt gcttggggaa cttgtgtccc taatgtgttt agattgctag attgctaagg 5280
   agctgatact ttgacagtgt ttttagacct gtgttactaa aaaaaagatg aatgtcctga 5340
   aaagggtgtt gggagggtgg ttcaacaaag aaacaaagat gttatggtgt ttagatttat 5400
   ggttgttaaa aatgtcatct caagtcaagt cactggtctg tttgcatttg atacattttt 5460
   gtactaacta gcattgtaaa attatttcat gattagaaat tacctgtgga tatttgtata 5520
   aaagtgtgaa ataaattttt tataaaagtg ttcattgttt cgtaacacag cattgtatat 5580
   gtgaagcaaa ctctaaaatt ataaatgaca acctgaatta tctatttcat caaaccaaag 5640
   ttcagtgttt ttatttttgg tgtctcatgt aatctcagat cagccaaaga tactagtgcc 5700
   aaagcaatgg gattcggggt ttttttctgt tttcgctcta tgtaggtgat cctcaagtct 5760
   ttcattttcc ttctttatga ttaaaagaaa cctacaggta tttaacaacc 5810
<210> 2
   <211> 1073
   <212> PRT
   <213> Homo sapiens
<400> 2
   Met Ala Ala Ala Gly Gln Leu Cys Leu Leu Tyr Leu Ser Ala Gly Leu
   1               5                   10                  15
   Leu Ser Arg Leu Gly Ala Ala Phe Asn Leu Asp Thr Arg Glu Asp Asn
               20                  25                  30
   Val Ile Arg Lys Tyr Gly Asp Pro Gly Ser Leu Phe Gly Phe Ser Leu
   35 40 45
   Ala Met His Trp Gln Leu Gln Pro Glu Asp Lys Arg Leu Leu Leu Val
   50 55 60
   Gly Ala Pro Arg Ala Glu Ala Leu Pro Leu Gln Arg Ala Asn Arg Thr
   65 70 75 80
   Gly Gly Leu Tyr Ser Cys Asp Ile Thr Ala Arg Gly Pro Cys Thr Arg
   85 90 95
   Ile Glu Phe Asp Asn Asp Ala Asp Pro Thr Ser Glu Ser Lys Glu Asp
   100 105 110
   Gln Trp Met Gly Val Thr Val Gln Ser Gln Gly Pro Gly Gly Lys Val
   115 120 125
   Val Thr Cys Ala His Arg Tyr Glu Lys Arg Gln His Val Asn Thr Lys
   130 135 140
   Gln Glu Ser Arg Asp Ile Phe Gly Arg Cys Tyr Val Leu Ser Gln Asn
   145 150 155 160
   Leu Arg Ile Glu Asp Asp Met Asp Gly Gly Asp Trp Ser Phe Cys Asp
   165 170 175
   Gly Arg Leu Arg Gly His Glu Lys Phe Gly Ser Cys Gln Gln Gly Val
   180 185 190
   Ala Ala Thr Phe Thr Lys Asp Phe His Tyr Ile Val Phe Gly Ala Pro
   195 200 205
   Gly Thr Tyr Asn Trp Lys Gly Ile Val Arg Val Glu Gln Lys Asn Asn
   210 215 220
   Thr Phe Phe Asp Met Asn Ile Phe Glu Asp Gly Pro Tyr Glu Val Gly
   225 230 235 240
   Gly Glu Thr Glu His Asp Glu Ser Leu Val Pro Val Pro Ala Asn Ser
   245 250 255
   Tyr Leu Gly Phe Ser Leu Asp Ser Gly Lys Gly Ile Val Ser Lys Asp
   260 265 270
   Glu Ile Thr Phe Val Ser Gly Ala Pro Arg Ala Asn His Ser Gly Ala
   275 280 285
   Val Val Leu Leu Lys Arg Asp Met Lys Ser Ala His Leu Leu Pro Glu
   290 295 300
   His Ile Phe Asp Gly Glu Gly Leu Ala Ser Ser Phe Gly Tyr Asp Val
   305 310 315 320
   Ala Val Val Asp Leu Asn Lys Asp Gly Trp Gln Asp Ile Val Ile Gly
   325 330 335
   Ala Pro Gln Tyr Phe Asp Arg Asp Gly Glu Val Gly Gly Ala Val Tyr
   340 345 350
   Val Tyr Met Asn Gln Gln Gly Arg Trp Asn Asn Val Lys Pro Ile Arg
   355 360 365
   Leu Asn Gly Thr Lys Asp Ser Met Phe Gly Ile Ala Val Lys Asn Ile
   370 375 380
   Gly Asp Ile Asn Gln Asp Gly Tyr Pro Asp Ile Ala Val Gly Ala Pro
   385 390 395 400
   Tyr Asp Asp Leu Gly Lys Val Phe Ile Tyr His Gly Ser Ala Asn Gly
   405 410 415
   Ile Asn Thr Lys Pro Thr Gln Val Leu Lys Gly Ile Ser Pro Tyr Phe
   420 425 430
   Gly Tyr Ser Ile Ala Gly Asn Met Asp Leu Asp Arg Asn Ser Tyr Pro
   435 440 445
   Asp Val Ala Val Gly Ser Leu Ser Asp Ser Val Thr Ile Phe Arg Ser
   450 455 460
   Arg Pro Val Ile Asn Ile Gln Lys Thr Ile Thr Val Thr Pro Asn Arg
   465 470 475 480
   Ile Asp Leu Arg Gln Lys Thr Ala Cys Gly Ala Pro Ser Gly Ile Cys
   485 490 495
   Leu Gln Val Lys Ser Cys Phe Glu Tyr Thr Ala Asn Pro Ala Gly Tyr
   500 505 510
   Asn Pro Ser Ile Ser Ile Val Gly Thr Leu Glu Ala Glu Lys Glu Arg
           515                 520                 525
   Arg Lys Ser Gly Leu Ser Ser Arg Val Gln Phe Arg Asn Gln Gly Ser
       530                 535                 540
   Glu Pro Lys Tyr Thr Gln Glu Leu Thr Leu Lys Arg Gln Lys Gln Lys
   545                 550                 555                 560
   Val Cys Met Glu Glu Thr Leu Trp Leu Gln Asp Asn Ile Arg Asp Lys
                   565                 570                 575
   Leu Arg Pro Ile Pro Ile Thr Ala Ser Val Glu Ile Gln Glu Pro Ser
               580                 585                 590
   Ser Arg Arg Arg Val Asn Ser Leu Pro Glu Val Leu Pro Ile Leu Asn
           595                 600                 605
   Ser Asp Glu Pro Lys Thr Ala His Ile Asp Val His Phe Leu Lys Glu
       610                 615                 620
   Gly Cys Gly Asp Asp Asn Val Cys Asn Ser Asn Leu Lys Leu Glu Tyr
   625                 630                 635                 640
   Lys Phe Cys Thr Arg Glu Gly Asn Gln Asp Lys Phe Ser Tyr Leu Pro
                   645                 650                 655
   Ile Gln Lys Gly Val Pro Glu Leu Val Leu Lys Asp Gln Lys Asp Ile
               660                 665                 670
   Ala Leu Glu Ile Thr Val Thr Asn Ser Pro Ser Asn Pro Arg Asn Pro
           675                 680                 685
   Thr Lys Asp Gly Asp Asp Ala His Glu Ala Lys Leu Ile Ala Thr Phe
       690                 695                 700
   Pro Asp Thr Leu Thr Tyr Ser Ala Tyr Arg Glu Leu Arg Ala Phe Pro
   705                 710                 715                 720
   Glu Lys Gln Leu Ser Cys Val Ala Asn Gln Asn Gly Ser Gln Ala Asp
                   725                 730                 735
   Cys Glu Leu Gly Asn Pro Phe Lys Arg Asn Ser Asn Val Thr Phe Tyr
               740                 745                 750
   Leu Val Leu Ser Thr Thr Glu Val Thr Phe Asp Thr Pro Asp Leu Asp
   755 760 765
   Ile Asn Leu Lys Leu Glu Thr Thr Ser Asn Gln Asp Asn Leu Ala Pro
   770 775 780
   Ile Thr Ala Lys Ala Lys Val Val Ile Glu Leu Leu Leu Ser Val Ser
   785 790 795 800
   Gly Val Ala Lys Pro Ser Gln Val Tyr Phe Gly Gly Thr Val Val Gly
   805 810 815
   Glu Gln Ala Met Lys Ser Glu Asp Glu Val Gly Ser Leu Ile Glu Tyr
   820 825 830
   Glu Phe Arg Val Ile Asn Leu Gly Lys Pro Leu Thr Asn Leu Gly Thr
   835 840 845
   Ala Thr Leu Asn Ile Gln Trp Pro Lys Glu Ile Ser Asn Gly Lys Trp
   850 855 860
   Leu Leu Tyr Leu Val Lys Val Glu Ser Lys Gly Leu Glu Lys Val Thr
   865 870 875 880
   Cys Glu Pro Gln Lys Glu Ile Asn Ser Leu Asn Leu Thr Glu Ser His
   885 890 895
   Asn Ser Arg Lys Lys Arg Glu Ile Thr Glu Lys Gln Ile Asp Asp Asn
   900 905 910
   Arg Lys Phe Ser Leu Phe Ala Glu Arg Lys Tyr Gln Thr Leu Asn Cys
   915 920 925
   Ser Val Asn Val Asn Cys Val Asn Ile Arg Cys Pro Leu Arg Gly Leu
   930 935 940
   Asp Ser Lys Ala Ser Leu Ile Leu Arg Ser Arg Leu Trp Asn Ser Thr
   945 950 955 960
   Phe Leu Glu Glu Tyr Ser Lys Leu Asn Tyr Leu Asp Ile Leu Met Arg
   965 970 975
   Ala Phe Ile Asp Val Thr Ala Ala Ala Glu Asn Ile Arg Leu Pro Asn
   980 985 990
   Ala Gly Thr Gln Val Arg Val Thr Val Phe Pro Ser Lys Thr Val Ala
                   995                 1000                1005
   Gln Tyr  Ser Gly Val Pro Trp  Trp Ile Ile Leu Val  Ala Ile Leu
       1010                 1015                 1020
   Ala Gly  Ile Leu Met Leu Ala  Leu Leu Val Phe Ile  Leu Trp Lys
       1025                 1030                 1035
   Cys Gly  Phe Phe Lys Arg Asn  Lys Lys Asp His Tyr  Asp Ala Thr
       1040                 1045                 1050
   Tyr His  Lys Ala Glu Ile His  Ala Gln Pro Ser Asp  Lys Glu Arg
       1055                 1060                 1065
   Leu Thr  Ser Asp Ala
       1070
<210> 3
   <211> 5680
   <212> DNA
   <213> Homo sapiens
<400> 3
   aacgggctca ttcagcggtc gcgagctgcc cgcgaggggg agcggccgga cggagagcgc    60
   gacccgtccc gggggtgggg ccgggcgcag cggcgagagg aggcgaaggt ggctgcggta   120
   gcagcagcgc ggcagcctcg gacccagccc ggagcgcagg gcggccgctg caggtccccg   180
   ctcccctccc cgtgcgtccg cccatggccg ccgccgggca gctgtgcttg ctctacctgt   240
   cggcggggct cctgtcccgg ctcggcgcag ccttcaactt ggacactcgg gaggacaacg   300
   tgatccggaa atatggagac cccgggagcc tcttcggctt ctcgctggcc atgcactggc   360
   aactgcagcc cgaggacaag cggctgttgc tcgtgggggc cccgcgggca gaagcgcttc   420
   cactgcagag agccaacaga acgggagggc tgtacagctg cgacatcacc gcccgggggc   480
   catgcacgcg gatcgagttt gataacgatg ctgaccccac gtcagaaagc aaggaagatc   540
   agtggatggg ggtcaccgtc cagagccaag gtccaggggg caaggtcgtg acatgtgctc   600
   accgatatga aaaaaggcag catgttaata cgaagcagga atcccgagac atctttgggc   660
   ggtgttatgt cctgagtcag aatctcagga ttgaagacga tatggatggg ggagattgga   720
   gcttttgtga tgggcgattg agaggccatg agaaatttgg ctcttgccag caaggtgtag   780
   cagctacttt tactaaagac tttcattaca ttgtatttgg agccccgggt acttataact   840
   ggaaagggat tgttcgtgta gagcaaaaga ataacacttt ttttgacatg aacatctttg   900
   aagatgggcc ttatgaagtt ggtggagaga ctgagcatga tgaaagtctc gttcctgttc   960
   ctgctaacag ttacttaggt ttttctttgg actcagggaa aggtattgtt tctaaagatg 1020
   agatcacttt tgtatctggt gctcccagag ccaatcacag tggagccgtg gttttgctga 1080
   agagagacat gaagtctgca catctcctcc ctgagcacat attcgatgga gaaggtctgg 1140
   cctcttcatt tggctatgat gtggcggtgg tggacctcaa caaggatggg tggcaagata 1200
   tagttattgg agccccacag tattttgata gagatggaga agttggaggt gcagtgtatg 1260
   tctacatgaa ccagcaaggc agatggaata atgtgaagcc aattcgtctt aatggaacca 1320
   aagattctat gtttggcatt gcagtaaaaa atattggaga tattaatcaa gatggctacc 1380
   cagatattgc agttggagct ccgtatgatg acttgggaaa ggtttttatc tatcatggat 1440
   ctgcaaatgg aataaatacc aaaccaacac aggttctcaa gggtatatca ccttattttg 1500
   gatattcaat tgctggaaac atggaccttg atcgaaattc ctaccctgat gttgctgttg 1560
   gttccctctc agattcagta actattttca gatcccggcc tgtgattaat attcagaaaa 1620
   ccatcacagt aactcctaac agaattgacc tccgccagaa aacagcgtgt ggggcgccta 1680
   gtgggatatg cctccaggtt aaatcctgtt ttgaatatac tgctaacccc gctggttata 1740
   atccttcaat atcaattgtg ggcacacttg aagctgaaaa agaaagaaga aaatctgggc 1800
   tatcctcaag agttcagttt cgaaaccaag gttctgagcc caaatatact caagaactaa 1860
   ctctgaagag gcagaaacag aaagtgtgca tggaggaaac cctgtggcta caggataata 1920
   tcagagataa actgcgtccc attcccataa ctgcctcagt ggagatccaa gagccaagct 1980
   ctcgtaggcg agtgaattca cttccagaag ttcttccaat tctgaattca gatgaaccca 2040
   agacagctca tattgatgtt cacttcttaa aagagggatg tggagacgac aatgtatgta 2100
   acagcaacct taaactagaa tataaatttt gcacccgaga aggaaatcaa gacaaatttt 2160
   cttatttacc aattcaaaaa ggtgtaccag aactagttct aaaagatcag aaggatattg 2220
   ctttagaaat aacagtgaca aacagccctt ccaacccaag gaatcccaca aaagatggcg 2280
   atgacgccca tgaggctaaa ctgattgcaa cgtttccaga cactttaacc tattctgcat 2340
   atagagaact gagggctttc cctgagaaac agttgagttg tgttgccaac cagaatggct 2400
   cgcaagctga ctgtgagctc ggaaatcctt ttaaaagaaa ttcaaatgtc actttttatt 2460
   tggttttaag tacaactgaa gtcacctttg acaccccaga tctggatatt aatctgaagt 2520
   tagaaacaac aagcaatcaa gataatttgg ctccaattac agctaaagca aaagtggtta 2580
   ttgaactgct tttatcggtc tcgggagttg ctaaaccttc ccaggtgtat tttggaggta 2640
   cagttgttgg cgagcaagct atgaaatctg aagatgaagt gggaagttta atagagtatg 2700
   aattcagggt aataaactta ggtaaacctc ttacaaacct cggcacagca accttgaaca 2760
   ttcagtggcc aaaagaaatt agcaatggga aatggttgct ttatttggtg aaagtagaat 2820
   ccaaaggatt ggaaaaggta acttgtgagc cacaaaagga gataaactcc ctgaacctaa 2880
   cggagtctca caactcaaga aagaaacggg aaattactga aaaacagata gatgataaca 2940
   gaaaattttc tttatttgct gaaagaaaat accagactct taactgtagc gtgaacgtga 3000
   actgtgtgaa catcagatgc ccgctgcggg ggctggacag caaggcgtct cttattttgc 3060
   gctcgaggtt atggaacagc acatttctag aggaatattc caaactgaac tacttggaca 3120
   ttctcatgcg agccttcatt gatgtgactg ctgctgccga aaatatcagg ctgccaaatg 3180
   caggcactca ggttcgagtg actgtgtttc cctcaaagac tgtagctcag tattcgggag 3240
   taccttggtg gatcatccta gtggctattc tcgctgggat cttgatgctt gctttattag 3300
   tgtttatact atggaagtgt ggattcttta aacgctctag gtacgatgac agtgttcccc 3360
   gataccatgc tgtaaggatc cggaaagaag agcgagagat caaagatgaa aagtatattg 3420
   ataaccttga aaaaaaacag tggatcacaa agtggaacga aaatgaaagc tactcatagc 3480
   gggggcctaa aaaaaaaaag cttcacagta cccaaactgc tttttccaac tcagaaattc 3540
   aatttggatt taaaagcctg ctcaatccct gaggactgat ttcagagtga ctacacacag 3600
   tacgaaccta cagttttaac tgtggatatt gttacgtagc ctaaggctcc tgttttgcac 3660
   agccaaattt aaaactgttg gaatggattt ttctttaact gccgtaattt aactttctgg 3720
   gttgccttta tttttggcgt ggctgactta catcatgtgt tggggaaggg cctgcccagt 3780
   tgcactcagg tgacatcctc cagatagtgt agctgaggag gcacctacac tcacctgcac 3840
   taacagagtg gccgtcctaa cctcgggcct gctgcgcaga cgtccatcac gttagctgtc 3900
   ccacatcaca agactatgcc attggggtag ttgtgtttca acggaaagtg ctgtcttaaa 3960
   ctaaatgtgc aatagaaggt gatgttgcca tcctaccgtc ttttcctgtt tcctagctgt 4020
   gtgaatacct gctcacgtca aatgcataca agtttcattc tccctttcac taaaacacac 4080
   aggtgcaaca gacttgaatg ctagttatac ttatttgtat atggtattta ttttttcttt    4140
   tctttacaaa ccattttgtt attgactaac aggccaaaga gtctccagtt tacccttcag    4200
   gttggtttaa tcaatcagaa ttagagcatg ggaggtcatc actttgacct aaattattta 4260
   ctgcaaaaag aaaatcttta taaatgtacc agagagagtt gttttaataa cttatctata 4320
   aactataacc tctccttcat gacagcctcc accccacaac ccaaaaggtt taagaaatag 4380
   aattataact gtaaagatgt ttatttcagg cattggatat tttttacttt agaagcctgc 4440
   ataatgtttc tggatttcat actgtaacat tcaggaattc ttggagaaaa tgggtttatt 4500
   cactgaactc tagtgcggtt tactcactgc tgcaaatact gtatattcag gacttgaaag 4560
   aaatggtgaa tgcctatggt ggatccaaac tgatccagta taagactact gaatctgcta     4620
   ccaaaacagt taatcagtga gtcgatgttc tattttttgt tttgtttcct cccctatctg      4680
   tattcccaaa aattactttg gggctaattt aacaagaact ttaaattgtg ttttaattgt     4740
   aaaaatggca gggggtggaa ttattactct atacattcaa cagagactga atagatatga     4800
   aagctgattt tttttaatta ccatgcttca caatgttaag ttatatgggg agcaacagca     4860
   aacaggtgct aatttgtttt ggatatagta taagcagtgt ctgtgttttg aaagaataga     4920
   acacagtttg tagtgccact gttgttttgg gggggctttt ttcttttcgg aaatcttaaa     4980
   ccttaagata ctaaggacgt tgttttggtt gtactttgga attcttagtc acaaaatata     5040
   ttttgtttac aaaaatttct gtaaaacagg ttataacagt gtttaaagtc tcagtttctt     5100
   gcttggggaa cttgtgtccc taatgtgttt agattgctag attgctaagg agctgatact     5160
   ttgacagtgt ttttagacct gtgttactaa aaaaaagatg aatgtcctga aaagggtgtt     5220
   gggagggtgg ttcaacaaag aaacaaagat gttatggtgt ttagatttat ggttgttaaa     5280
   aatgtcatct caagtcaagt cactggtctg tttgcatttg atacattttt gtactaacta     5340
   gcattgtaaa attatttcat gattagaaat tacctgtgga tatttgtata aaagtgtgaa     5400
   ataaattttt tataaaagtg ttcattgttt cgtaacacag cattgtatat gtgaagcaaa     5460
   ctctaaaatt ataaatgaca acctgaatta tctatttcat caaaccaaag ttcagtgttt     5520
   ttatttttgg tgtctcatgt aatctcagat cagccaaaga tactagtgcc aaagcaatgg     5580
   gattcggggt ttttttctgt tttcgctcta tgtaggtgat cctcaagtct ttcattttcc     5640
   ttctttatga ttaaaagaaa cctacaggta tttaacaacc                           5680
<210> 4
   <211> 1091
   <212> PRT
   <213> Homo sapiens
<400> 4
   Met Ala Ala Ala Gly Gln Leu Cys Leu Leu Tyr Leu Ser Ala Gly Leu
   1               5                   10                  15
   Leu Ser Arg Leu Gly Ala Ala Phe Asn Leu Asp Thr Arg Glu Asp Asn
               20                  25                  30
   Val Ile Arg Lys Tyr Gly Asp Pro Gly Ser Leu Phe Gly Phe Ser Leu
           35                  40                  45
   Ala Met His Trp Gln Leu Gln Pro Glu Asp Lys Arg Leu Leu Leu Val
   50 55 60
   Gly Ala Pro Arg Ala Glu Ala Leu Pro Leu Gln Arg Ala Asn Arg Thr
   65 70 75 80
   Gly Gly Leu Tyr Ser Cys Asp Ile Thr Ala Arg Gly Pro Cys Thr Arg
   85 90 95
   Ile Glu Phe Asp Asn Asp Ala Asp Pro Thr Ser Glu Ser Lys Glu Asp
   100 105 110
   Gln Trp Met Gly Val Thr Val Gln Ser Gln Gly Pro Gly Gly Lys Val
   115 120 125
   Val Thr Cys Ala His Arg Tyr Glu Lys Arg Gln His Val Asn Thr Lys
   130 135 140
   Gln Glu Ser Arg Asp Ile Phe Gly Arg Cys Tyr Val Leu Ser Gln Asn
   145 150 155 160
   Leu Arg Ile Glu Asp Asp Met Asp Gly Gly Asp Trp Ser Phe Cys Asp
   165 170 175
   Gly Arg Leu Arg Gly His Glu Lys Phe Gly Ser Cys Gln Gln Gly Val
   180 185 190
   Ala Ala Thr Phe Thr Lys Asp Phe His Tyr Ile Val Phe Gly Ala Pro
   195 200 205
   Gly Thr Tyr Asn Trp Lys Gly Ile Val Arg Val Glu Gln Lys Asn Asn
   210 215 220
   Thr Phe Phe Asp Met Asn Ile Phe Glu Asp Gly Pro Tyr Glu Val Gly
   225 230 235 240
   Gly Glu Thr Glu His Asp Glu Ser Leu Val Pro Val Pro Ala Asn Ser
   245 250 255
   Tyr Leu Gly Phe Ser Leu Asp Ser Gly Lys Gly Ile Val Ser Lys Asp
   260 265 270
   Glu Ile Thr Phe Val Ser Gly Ala Pro Arg Ala Asn His Ser Gly Ala
   275 280 285
   Val Val Leu Leu Lys Arg Asp Met Lys Ser Ala His Leu Leu Pro Glu
   290 295 300
   His Ile Phe Asp Gly Glu Gly Leu Ala Ser Ser Phe Gly Tyr Asp Val
   305 310 315 320
   Ala Val Val Asp Leu Asn Lys Asp Gly Trp Gln Asp Ile Val Ile Gly
   325 330 335
   Ala Pro Gln Tyr Phe Asp Arg Asp Gly Glu Val Gly Gly Ala Val Tyr
   340 345 350
   Val Tyr Met Asn Gln Gln Gly Arg Trp Asn Asn Val Lys Pro Ile Arg
   355 360 365
   Leu Asn Gly Thr Lys Asp Ser Met Phe Gly Ile Ala Val Lys Asn Ile
   370 375 380
   Gly Asp Ile Asn Gln Asp Gly Tyr Pro Asp Ile Ala Val Gly Ala Pro
   385 390 395 400
   Tyr Asp Asp Leu Gly Lys Val Phe Ile Tyr His Gly Ser Ala Asn Gly
   405 410 415
   Ile Asn Thr Lys Pro Thr Gln Val Leu Lys Gly Ile Ser Pro Tyr Phe
   420 425 430
   Gly Tyr Ser Ile Ala Gly Asn Met Asp Leu Asp Arg Asn Ser Tyr Pro
   435 440 445
   Asp Val Ala Val Gly Ser Leu Ser Asp Ser Val Thr Ile Phe Arg Ser
   450 455 460
   Arg Pro Val Ile Asn Ile Gln Lys Thr Ile Thr Val Thr Pro Asn Arg
   465 470 475 480
   Ile Asp Leu Arg Gln Lys Thr Ala Cys Gly Ala Pro Ser Gly Ile Cys
   485 490 495
   Leu Gln Val Lys Ser Cys Phe Glu Tyr Thr Ala Asn Pro Ala Gly Tyr
   500 505 510
   Asn Pro Ser Ile Ser Ile Val Gly Thr Leu Glu Ala Glu Lys Glu Arg
   515 520 525
   Arg Lys Ser Gly Leu Ser Ser Arg Val Gln Phe Arg Asn Gln Gly Ser
   530 535 540
   Glu Pro Lys Tyr Thr Gln Glu Leu Thr Leu Lys Arg Gln Lys Gln Lys
   545 550 555 560
   Val Cys Met Glu Glu Thr Leu Trp Leu Gln Asp Asn Ile Arg Asp Lys
   565 570 575
   Leu Arg Pro Ile Pro Ile Thr Ala Ser Val Glu Ile Gln Glu Pro Ser
   580 585 590
   Ser Arg Arg Arg Val Asn Ser Leu Pro Glu Val Leu Pro Ile Leu Asn
   595 600 605
   Ser Asp Glu Pro Lys Thr Ala His Ile Asp Val His Phe Leu Lys Glu
   610 615 620
   Gly Cys Gly Asp Asp Asn Val Cys Asn Ser Asn Leu Lys Leu Glu Tyr
   625 630 635 640
   Lys Phe Cys Thr Arg Glu Gly Asn Gln Asp Lys Phe Ser Tyr Leu Pro
   645 650 655
   Ile Gln Lys Gly Val Pro Glu Leu Val Leu Lys Asp Gln Lys Asp Ile
   660 665 670
   Ala Leu Glu Ile Thr Val Thr Asn Ser Pro Ser Asn Pro Arg Asn Pro
   675 680 685
   Thr Lys Asp Gly Asp Asp Ala His Glu Ala Lys Leu Ile Ala Thr Phe
   690 695 700
   Pro Asp Thr Leu Thr Tyr Ser Ala Tyr Arg Glu Leu Arg Ala Phe Pro
   705 710 715 720
   Glu Lys Gln Leu Ser Cys Val Ala Asn Gln Asn Gly Ser Gln Ala Asp
   725 730 735
   Cys Glu Leu Gly Asn Pro Phe Lys Arg Asn Ser Asn Val Thr Phe Tyr
   740 745 750
   Leu Val Leu Ser Thr Thr Glu Val Thr Phe Asp Thr Pro Asp Leu Asp
   755 760 765
   Ile Asn Leu Lys Leu Glu Thr Thr Ser Asn Gln Asp Asn Leu Ala Pro
   770 775 780
   Ile Thr Ala Lys Ala Lys Val Val Ile Glu Leu Leu Leu Ser Val Ser
   785 790 795 800
   Gly Val Ala Lys Pro Ser Gln Val Tyr Phe Gly Gly Thr Val Val Gly
   805 810 815
   Glu Gln Ala Met Lys Ser Glu Asp Glu Val Gly Ser Leu Ile Glu Tyr
   820 825 830
   Glu Phe Arg Val Ile Asn Leu Gly Lys Pro Leu Thr Asn Leu Gly Thr
   835 840 845
   Ala Thr Leu Asn Ile Gln Trp Pro Lys Glu Ile Ser Asn Gly Lys Trp
   850 855 860
   Leu Leu Tyr Leu Val Lys Val Glu Ser Lys Gly Leu Glu Lys Val Thr
   865 870 875 880
   Cys Glu Pro Gln Lys Glu Ile Asn Ser Leu Asn Leu Thr Glu Ser His
   885 890 895
   Asn Ser Arg Lys Lys Arg Glu Ile Thr Glu Lys Gln Ile Asp Asp Asn
   900 905 910
   Arg Lys Phe Ser Leu Phe Ala Glu Arg Lys Tyr Gln Thr Leu Asn Cys
   915 920 925
   Ser Val Asn Val Asn Cys Val Asn Ile Arg Cys Pro Leu Arg Gly Leu
   930 935 940
   Asp Ser Lys Ala Ser Leu Ile Leu Arg Ser Arg Leu Trp Asn Ser Thr
   945 950 955 960
   Phe Leu Glu Glu Tyr Ser Lys Leu Asn Tyr Leu Asp Ile Leu Met Arg
   965 970 975
   Ala Phe Ile Asp Val Thr Ala Ala Ala Glu Asn Ile Arg Leu Pro Asn
   980 985 990
   Ala Gly Thr Gln Val Arg Val Thr Val Phe Pro Ser Lys Thr Val Ala
   995 1000 1005
   Gln Tyr Ser Gly Val Pro Trp Trp Ile Ile Leu Val Ala Ile Leu
           1010                1015                1020
   Ala Gly  Ile Leu Met Leu Ala  Leu Leu Val Phe Ile  Leu Trp Lys
       1025                1030                1035
   Cys Gly  Phe Phe Lys Arg Ser  Arg Tyr Asp Asp Ser  Val Pro Arg
       1040                1045                1050
   Tyr His  Ala Val Arg Ile Arg  Lys Glu Glu Arg Glu  Ile Lys Asp
       1055                1060                1065
   Glu Lys  Tyr Ile Asp Asn Leu  Glu Lys Lys Gln Trp  Ile Thr Lys
       1070                1075                1080
   Trp Asn  Glu Asn Glu Ser Tyr  Ser
       1085                1090
<210> 5
   <211> 28
   <212> DNA
   <213> Artificial Sequence
<220>
   <223> Oligonucleotide primer
<400> 5
   cggggtacct tggaagaggg aggaggag 28
<210> 6
   <211> 26
   <212> DNA
   <213> Artificial Sequence
<220>
   <223> Oligonucleotide primer
<400> 6
   gaagatctag tcactcgccg ctggca 26
<210> 7
   <211> 20
   <212> DNA
   <213> Artificial Sequence
<220>
   <223> Oligonucleotide primer
<400> 7
   ctaacggagt ctcacaactc 20
<210> 8
   <211> 19
   <212> DNA
   <213> Artificial Sequence
<220>
   <223> Oligonucleotide primer
<400> 8
   agttaaaact gtaggttcg 19
<210> 9
   <211> 20
   <212> DNA
   <213> Artificial Sequence
<220>
   <223> Oligonucleotide primer
<400> 9
   tgctgaaaga aaataccaga 20
<210> 10
   <211> 28
   <212> DNA
   <213> Artificial Sequence
<220>
   <223> Oligonucleotide primer
<400> 10
   gctctagaga aaaagcagtt tgggtact 28
<210> 11
   <211> 19
   <212> DNA
   <213> Artificial Sequence
<220>
   <223> Oligonucleotide primer
<400> 11
   tgggatatgc ctccaggtt 19
<210> 12
   <211> 20
   <212> DNA
   <213> Artificial Sequence
<220>
   <223> Oligonclueotide promer
<400> 12
   tgtagccaca gggtttcctc 20
<210> 13
   <211> 22
   <212> DNA
   <213> Artificial Sequence
<220>
   <223> Sh oligonucleotide
<400> 13
   gatcattatg atgccacata tc 22

## Description

### CROSS-REFERENCE TO RELATED APPLICATION

This application claims priority on U.S. applications 61/121,337 filed December 10, 2008 and 61/177,398 filed May 12, 2009.

This application contains a sequence listing submitted herewith electronically. The content of the sequence listing is incorporated by reference in this application.

### BACKGROUND

Integrins are a family of heterodimeric transmembrane receptors that, besides providing a physical link between the basement membrane (BM) and the cytoskeleton of epithelial cells, act as platforms for intracellular signaling as a consequence of ligand binding and cross talk with receptor tyrosine-kinases (RTKs) (Giancotti and Tarone, 2003). To date, 18 α and 8 β subunits have been identified in the human, leading to the formation of at least 24 distinct functional receptors. However, extensive alternative splicing and post-translational modification of both groups of subunits leads to the generation of considerably more forms *in vivo* (de Melker and Sonnenberg, 1999).

The α6 subunit mRNA undergoes alternative splicing yielding two distinct isoforms (Hogervorst et al., 1991), termed α6A and α6B, with distinct cytoplasmic domains and dissimilar patterns of expression throughout the human organism (Hogervorst et al., 1993). The different isoforms result from alternative splicing of a single exon (Hogervorst et al., 1991). Thus, the inclusion of an alternatively spliced exon results in the formation of the α6A variant, while exclusion leads to a reading frame shift, usage of an alternative stop codon and formation of the α6B variant. The A variant has been reported to be the only variant expressed in the mammary gland, peripheral nerves and basal keratinocytes while the B variant is predominant in the kidney. The intestine was initially reported to express both variants (Hogervorst et al., 1993). These patterns of expression for α6A and α6B as well as their dissimilar temporal expression during embryonic development (Thorsteinsdottir et al., 1995) may imply that they serve different biological functions. The α6A and -B subunits possess divergent capacities of initiating intracellular biochemical events, namely tyrosine phosphorylation of paxillin (Shaw et al., 1995) and activation of the Ras-MEK-ERK pathway (Wei et al., 1998).

In the human intestine, the α6 subunit dimerizes with the β4 subunit forming the α6β4 integrin (Basora et al., 1999). The relatively simple structural and functional renewal unit of the small intestine, the crypt-villus axis, makes it an attractive model for the study of epithelial cell proliferation and maturation (Babyatsky and Podolsky, 1995). Positional control of the enterocytes and their subsequent function is controlled by cell-cell and cell-extracellular matrix (ECM) interactions with the underlying the basement membrane (BM) (Teller and Beaulieu, 2001). The importance of the latter is exemplified by the inductive effects on enterocytic cytology by specific laminin variants (Vachon and Beaulieu, 1995; Virtanen et al., 2000), while analysis of several molecules involved in cell-ECM interactions, including integrins, has revealed distinct patterns of expression along the crypt-villus axis in relation to the differentiation state of enterocytes (Beaulieu, 1997; Teller and Beaulieu, 2001). Furthermore, the α6β4 integrin has been shown to be an important player in mediating migration and invasion of colon cancer cells (Lohi et al., 2000; Mercurio and Rabinovitz, 2001; Ni et al., 2005; Pouliot et al., 2001).

Integrins do not possess intrinsic signaling capacities, but rather mediate positional information by interacting with a large range of scaffolding proteins resulting in activation of several signaling molecules, such as Ras and PI3K, leading to subsequent activation of, among other molecules, JNK, Jun, Erk and CyclinD (Giancotti et al., 2003). The net result of this integrin mediated intracellular signaling is control of cellular functions such as proliferation, migration, invasion and survival, all of which are pivotal events in cancer progression (Guo and Giancotti, 2004).

The accumulated findings of an association between high expression levels of the α6 integrin subunit and carcinoma cell invasion, metastatic capacity, apoptosis evasion and negative patient outcome (Mercurio and Rabinovitz, 2001; Friedrichs et al., 1995; Chung and Mercurio, 2004) strongly argues in favor of a role for α6 containing integrins in human cancers. While the α6 subunit can dimerize with either β1 or β4 subunits, it preferentially dimerizes with the β4 subunit. In fact, in cells that express significant amounts of β4, such as human intestinal epithelial cells (Basora et al., 1999), the formation of α6β1 is nominal. Recent work has demonstrated an overall up-regulation of the expression of the β4 integrin subunit in primary tumors of the human colon (Ni et al., 2005) strongly supporting the notion that the α6β4 integrin is an important player in the migration and invasion of colon cancer cells (Mercurio and Rabinovitz, 2001; Pouliot et al., 2001). These observations, taken together with the reported presence of this major laminin receptor at the invasive front of colorectal cancers (Lohi et al, 2000), argues for an important role for the α6β4 integrin in colon cancer progression (Mercurio et al., 2001).

It would be highly desirable to be provided with the relationship between α6A and α6B isoforms and the proliferation of normal and cancer cells. This relationship would be beneficial in the design of new diagnostic and therapeutic tools for conditions associated with aberrant proliferation, such as neoplastic proliferation.

### BRIEF SUMMARY

As shown herein, the B:A ratio of the isoforms of the integrin α6 are tightly linked to the regulation of the cell cycle. Therefore, the present application concerns the modulation of that ratio for diagnosing an hyper or hypoproliferative state.

The present application provides a method of diagnosing a hyperproliferative state in an individual. The method comprises determining a ratio between an isoform B and an isoform A of an integrin α6 subunit in a cell from said individual, wherein a ratio being lower than a control ratio is indicative of the presence of the hyperproliferative state in said individual. In an embodiment, the ratio between the isoform B and the isoform A of the integrin α6 subunit is determined by quantifying the mRNA specific for the isoform B and the mRNA specific for the isoform A in said cell. In still another embodiment, the determination can be done by PCR and/or real-time PCR. In another embodiment, wherein the ratio between the isoform B and the isoform A of the integrin α6 subunit is determined by quantifying the polypeptide specific for the isoform B and the polypeptide specific for the isoform A in said cell. In yet another embodiment, the determination can be done with an antibody and/or an ELISA assay. In an embodiment, the control ratio is a ratio between the isoform B and the isoform A of an integrin α6 subunit in a cell from a healthy control patient or a healthy tissue free of hyperproliferation (e.g. abnormally elevated proliferation). In still another embodiment, the control ratio is of about 1.5. In yet another embodiment, the hyperproliferative state is cancer, such as a cancer associated with a gastro-intestinal tract, a carcinoma and/or a colon cancer.

Specifically, in accordance to the present invention, the hyperproliferative state is a colon cancer, and even more specifically, the colon cancer is a carcinoma.

### BRIEF DESCRIPTION OF THE DRAWINGS

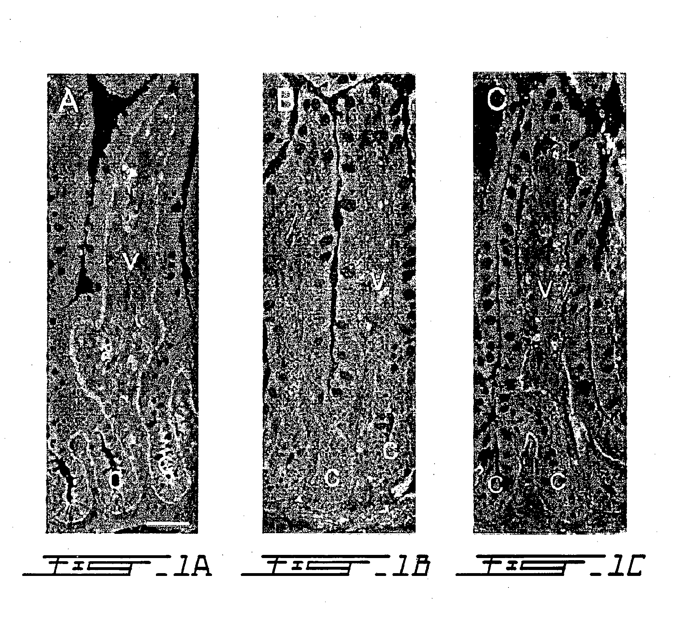

**Figure 1****.** Representative immunofluorescent staining on tissue sections of the adult small intestinal mucosa for detection of the α6 integrin subunit and its α6A and α6B splice variants. (A) A common α6 epitope was detected at the base of both crypt (c) and villus (v) cells. (B) The A variant was predominantly located in the crypts (c). (C) The B variant was detected in the villus (v) and upper and lower thirds of the crypt (c). Red-brown signal: Evan blue counter stain. Magnifications: A-C: scale bar in A = 50 µm.

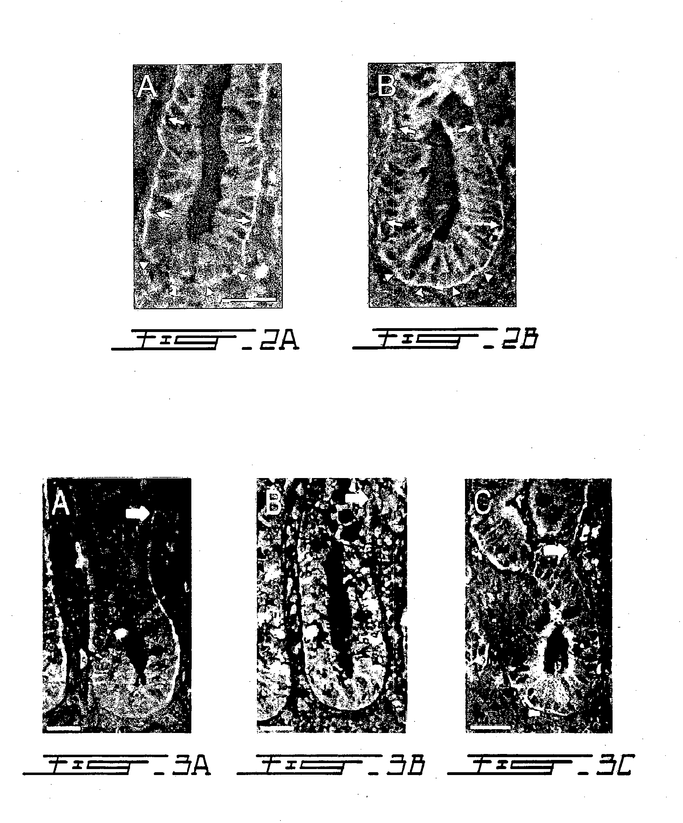

**Figure 2****.** Representative immunofluorescence staining of the crypt region stained for the detection of the α6A (A) and α6B (B) showing the A variant in the proliferative zone (arrows) and the B variant in the Paneth cell reion (arrowheads). Scale bar = 25 µm.
**Figure 3****.** Representative double immunofluorescent staining on tissue sections of adult small intestinal crypts for detection of α6A (A), Ki67 (B), α6B (C) in green and lysozyme in red (A-C). Predominant distribution of the α6A subunit was found in the middle part of the crypt (A), above the Paneth cell region as determined with lysozyme immunostaining and adjacent to the Ki67-positive region as determined in the corresponding crypt from a serial cryosection (B). The α6B subunit was found to be predominant in the upper crypt/lower villus region as well as in the bottom of the glands (C), a region that contains Paneth cells as identified with lysozyme. Scale bars = 25 µm.

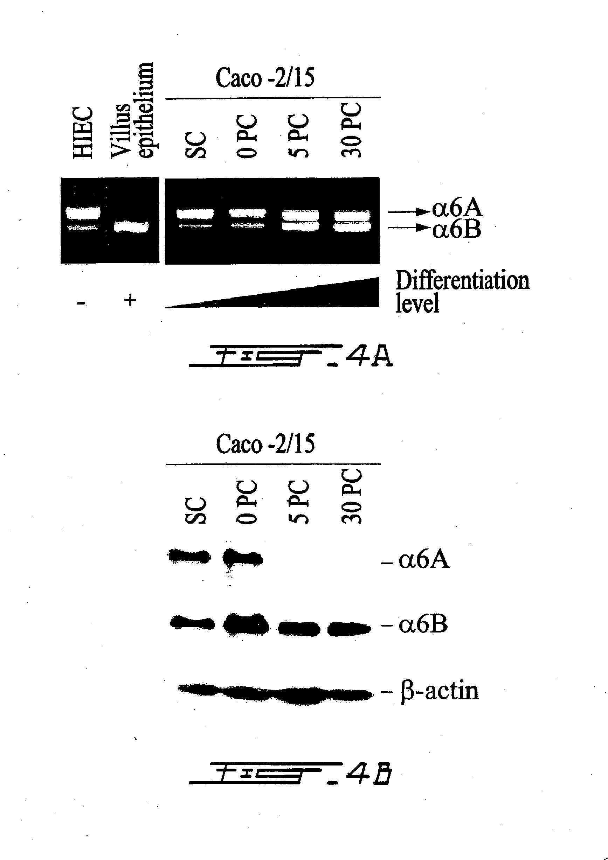

**Figure 4****.** Competitive RT-PCR of splice variant expression and western blot analysis showing down-regulation of the α6A variant upon cell-cycle exit in intestinal cells. (A) Representative competitive RT-PCR results of the expression of the α6A and α6B isoforms in HIEC: proliferative crypt cells; Villus epithelium: extracts of differentiated human villus cells; Caco-2/15: proliferative at sub-confluence (SC) while differentiating at various intervals (days) post-confluence (PC). (B) Representative western blot analysis of the α6A and α6B isoforms proteins in Caco-2/1 proliferative cells at sub-confluence (SC) while differentiating at various intervals (days)post-confluence (PC).

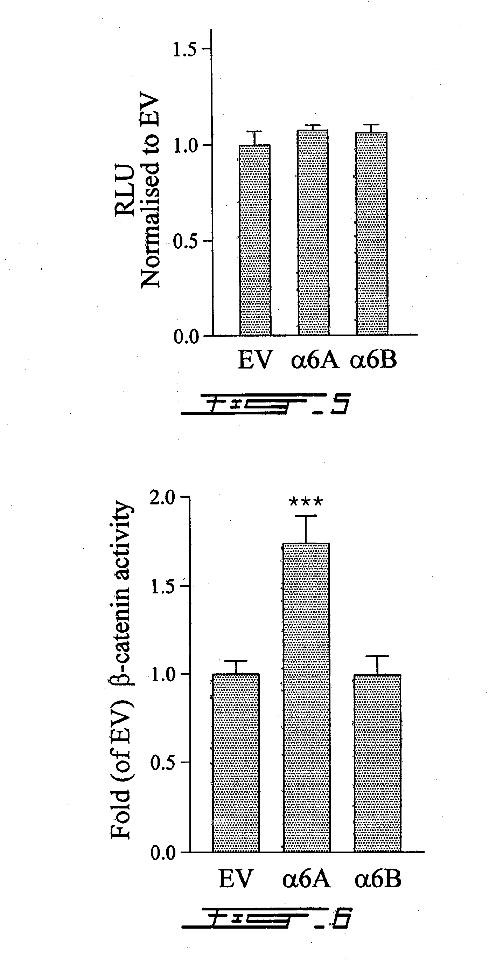

**Figure 5****.** Representative experiment of the promoter activity of the intestinal differentiation marker DPPIV when co-transfected with either α6A or α6B expression vectors in 95-100% confluent Caco-2/15 cells. Empty vector (EV) was used as control. Mean ± SEM.
**Figure 6****.** Response of promoter activities associated with proliferation. Representative experiment of β-catenin/TCF promoter activity in response to co-transfection with either α6A or α6B expression vectors in 40-60% confluent Caco-2/15 cells. Empty vector (EV) was used as control. Mean ± SEM. ***: statistically significantly different from EV, p < 0.001, Tukey's One Way Analysis of Variance (ANOVA).

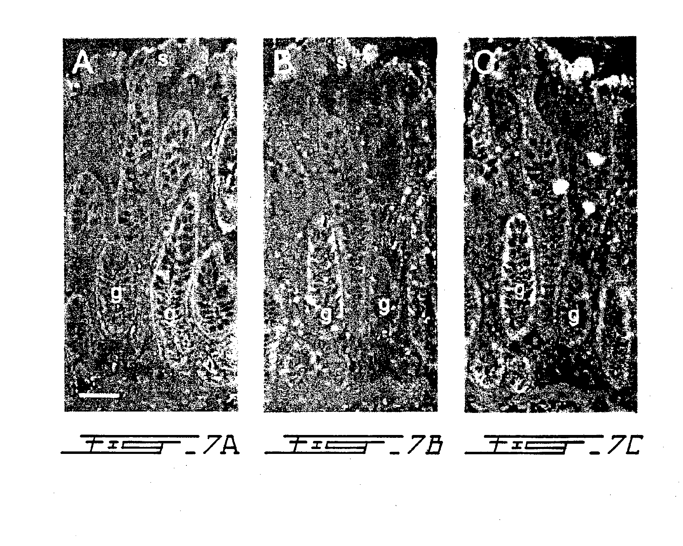

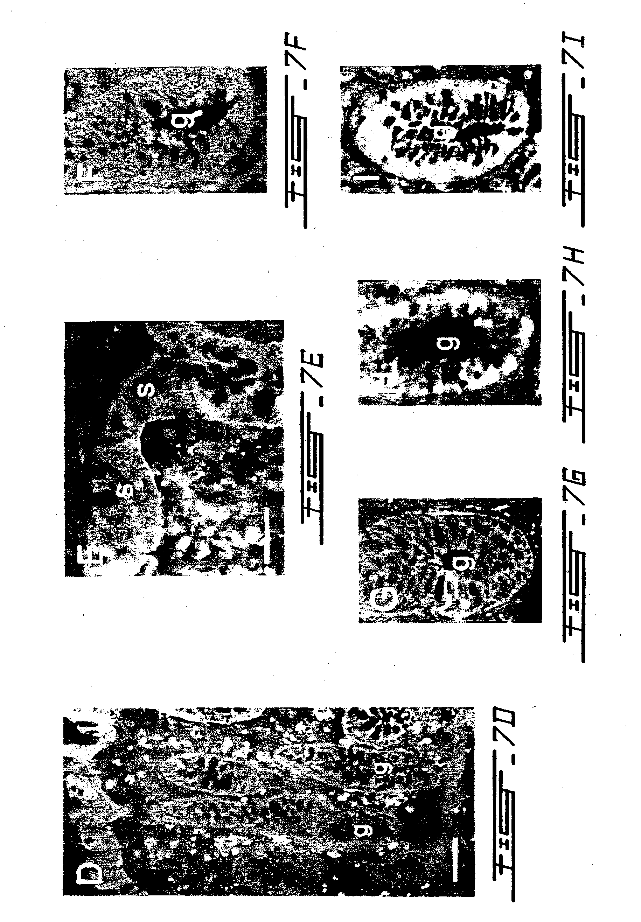

**Figure 7****.** Representative immunofluorescent staining of frozen serial sections of the adult colonic mucosa for the detection of the α6A and α6B splice variants and proliferative markers. (A) Expression of the A variant showing a predominant distribution in the lower half of the glands (g), the region containing the progenitor cells as confirmed by immunodetection with Ki67 (B) and Rbm19 (C). (D, E) The B variant was detected in the upper half of the glands and at the surface (s) epithelium. (F-I) Higher magnification of the lower gland region stained for the detection of α6B (F), α6A (G), Ki67 (H) and Rbm19 (I) showed that in the colon, α6B is absent from the lower crypt while both α6A and the proliferative zone extend to the bottom of the glands. Red-brown signal: Evan blue counter stain. Magnifications: A-D: scale bars in A and D = 50 µm; E-I: scale bar in E = 25 µm.

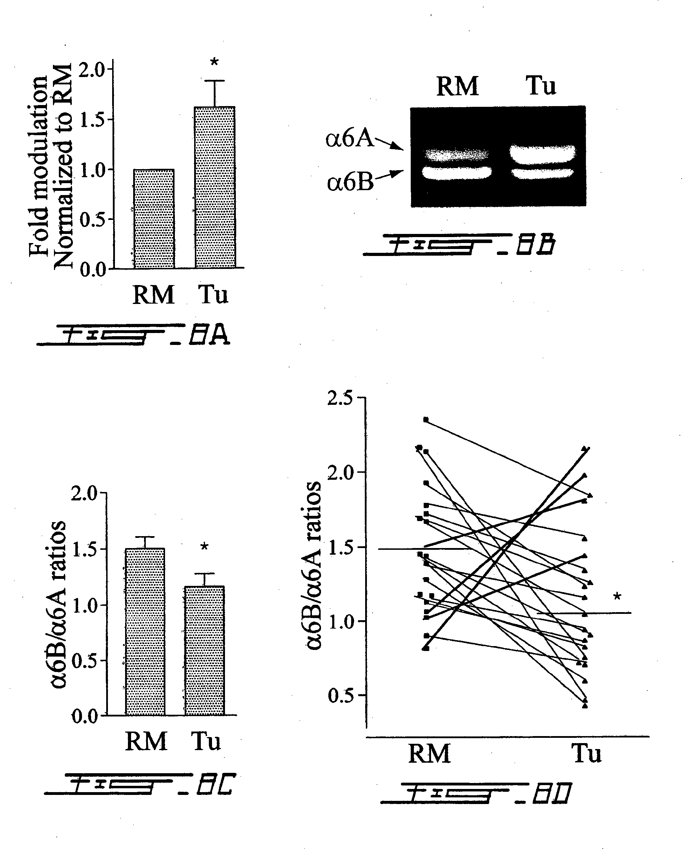

**Figure 8****.** Integrin α6 is up-regulated in colon cancer cells and undergoes a shift towards the α6A variant. (A) Quantitative RT-PCR of total α6 subunit mRNA in patient matched resection margins (RM) and corresponding primary tumors (Tu). Mean ± SEM. *: p = 0.025, n = 21, paired t-test. Representative competitive RT-PCR of the α6A and α6B variants (B) and (C) ratio of α6B/α6A transcript levels in patient matched RM and primary tumors of the human colon. Mean ± SEM. *: p = 0.05, n = 21, paired t-test. (D) Dot graphs of the individual α6B/α6A ratios showing a sharp decrease in 17 (grey) of the 21 paired samples analyzed.

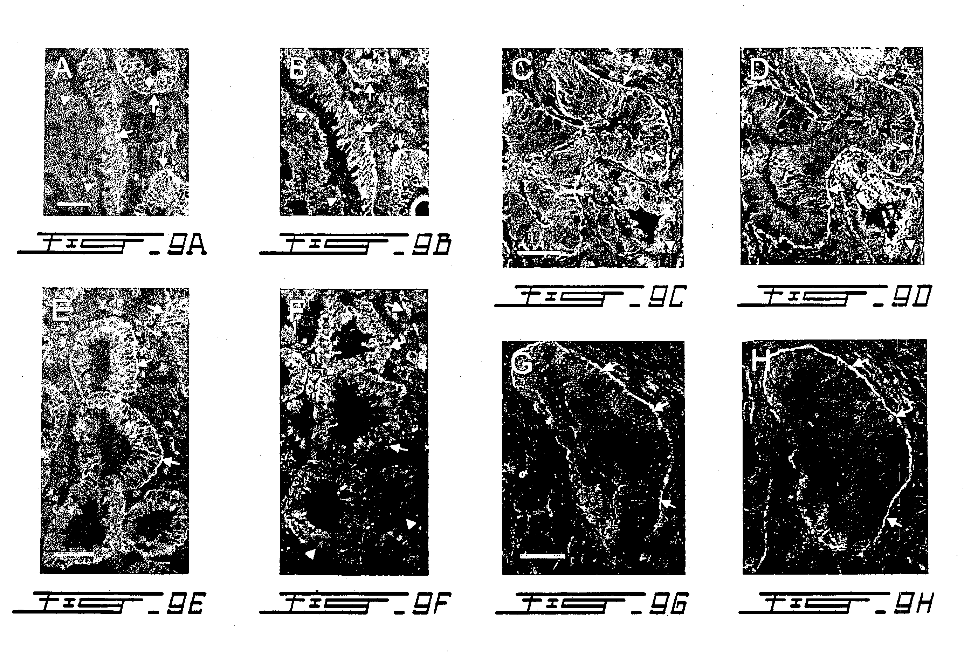

**Figure 9****.** Expression of α6A and α6B integrin subunits in colon cancer specimens. Representative images by indirect immunofluorescence staining of α6A (A, C, E and G) and α6B (B, D, F and H) integrin subunits in serial sections of colon cancer specimens (A, B, C, D, E, F and G, H) showing the loss of variant segregation, leading to widespread overlap of both variants (arrows). Some regions, however, displayed the expression of only one variant, with no expression of the other (arrowheads). Red-brown signal: Evan's blue counter stain. Magnifications: scale bars = 50 µm.

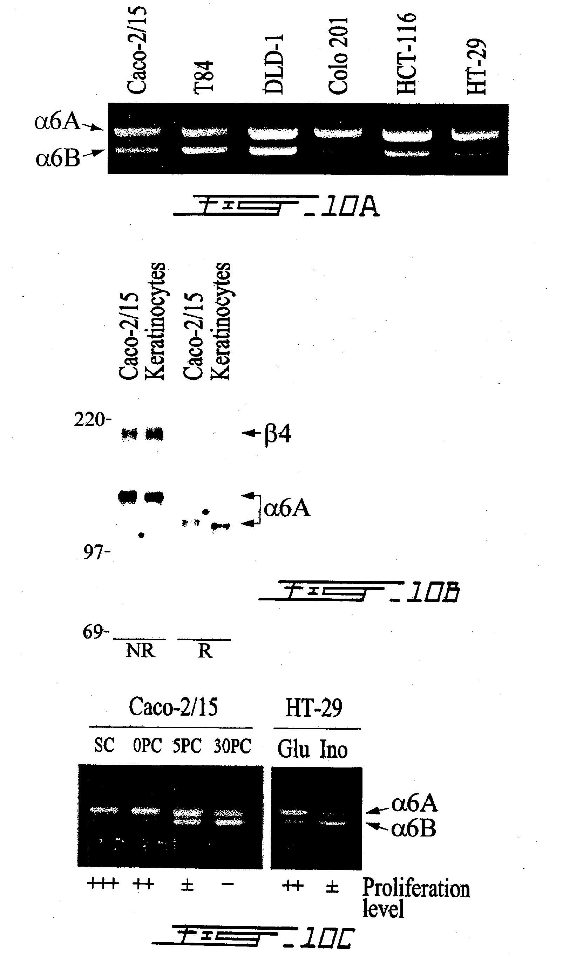

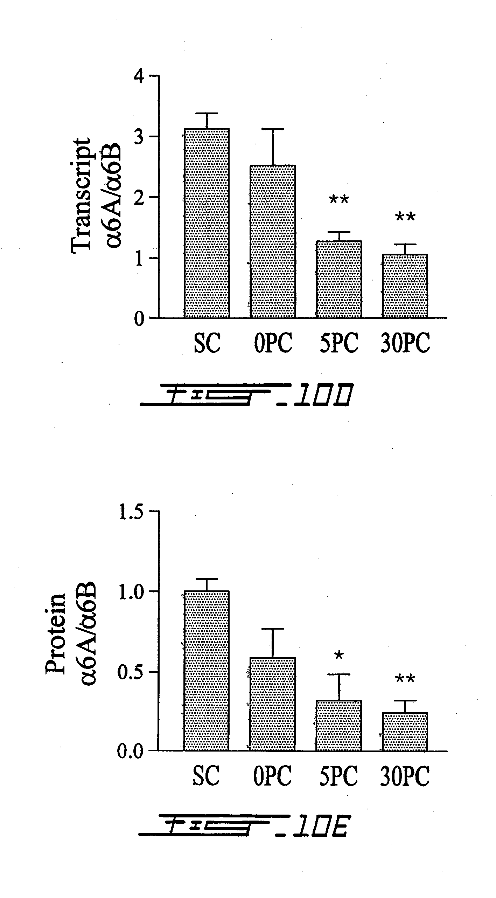

**Figure 10****.** Analysis of α6A and α6B integrin subunits in colon cancer cell models. (A) Representative competitive RT-PCR of the α6A and α6B variants in six colon cancer cell lines. (B) Immunoprecipitation of the α6 subunits using the G0H3 antibody from metabolically labelled Caco-2/15 cells and keratinocytes and analyzed on SDS-PAGE under non-reduced (NR) and reduced (R) conditions showing that α6 predominantly associates with the β4 subunit in these cells. Apparent molecular weights are indicated on the left side. The dots indicate the expected sites of β1 subunit migration under both NR and R conditions. (C) Further analysis by competitive RT-PCR for splice variant expression of two intestinal cell lines at different differentiation stages showing up-regulation of the α6B variant upon cell-cycle exit. Caco-2/15: proliferative at sub-confluence (SC) while becoming non-proliferative at post-confluence (PC); HT-29: grown under non-permissive (Glu: glucose) or permissive (Ino: inosine) conditions for differentiation and cell cycle exit. (D, E) The α6A/α6B ratio of transcript (D) and protein (E) levels relative to differentiation of the various intestinal cells. Means ± SEM, n = 3-6. *, **: Statistically significantly different with p < 0.05 and 0.01, respectively, from sub-confluent Caco-2/15. Tukey's One Way Analysis of Variance (ANOVA).

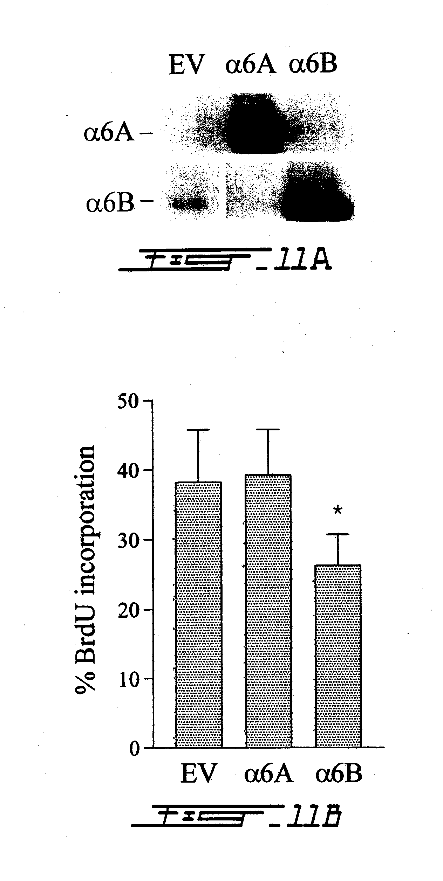

**Figure 11****.** Forced expression of the α6B subunit inhibits intestinal cell proliferation. (A) Forced expression of the α6A and α6B integrin subunits was confirmed by western blot. (B) Quantification of Caco-2/15 cells undergoing S phase entry expressing α6A and α6B vs an empty vector control was assessed by BrdU incorporation. Mean ±SD. *: Statistically significantly different from the A variant, p < 0.01, Tukey's One Way Analysis of Variance (ANOVA).

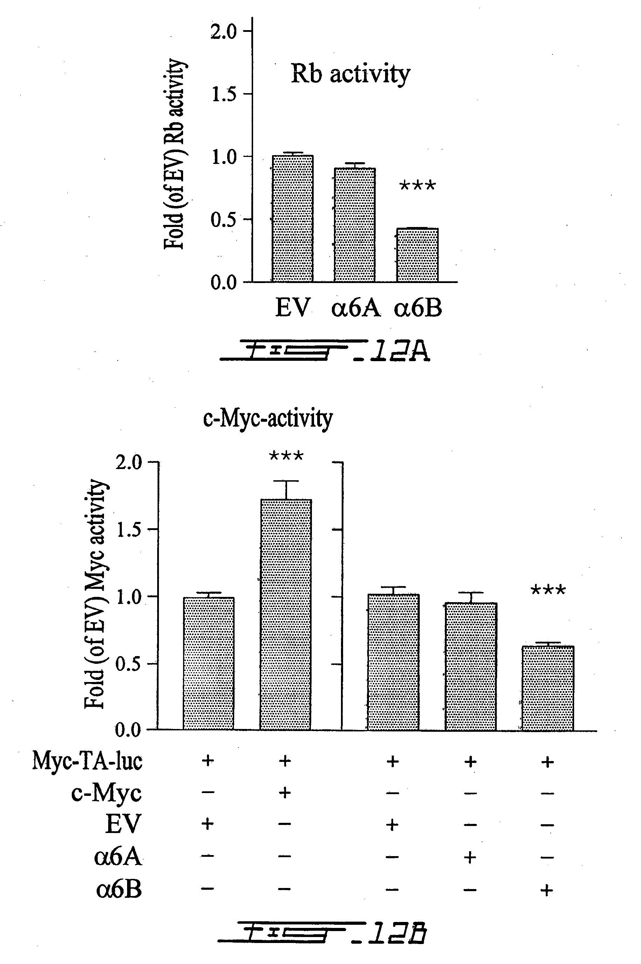

**Figure 12****.** Response of Rb and c-Myc promoter activities in Caco-2/15 cells. (A) Rb activity was determined with an Rb-TA-Luc responsive promoter in the presence of forced α6A or α6B expression. (B) c-Myc activity was determined with a Myc-TA-luc responsive promoter. Left: Effect of forced c-Myc expression on the myc-responsive promoter. Right: Effect of forced α6A or α6B expression on the myc-responsive promoter. Mean ± SEM. ***: statistically significantly different from empty vector (EV), p < 0.001, Tukey's One Way Analysis of Variance (ANOVA). (C) Representative western blot for c-Myc expression in Caco-2/15 cells stably expressing the α6A and α6B integrin subunit or EV as in Figure 8.

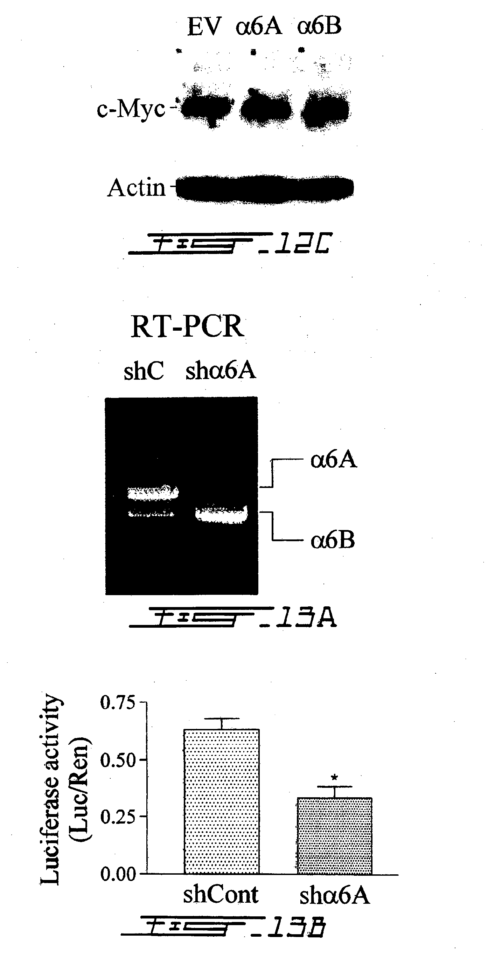

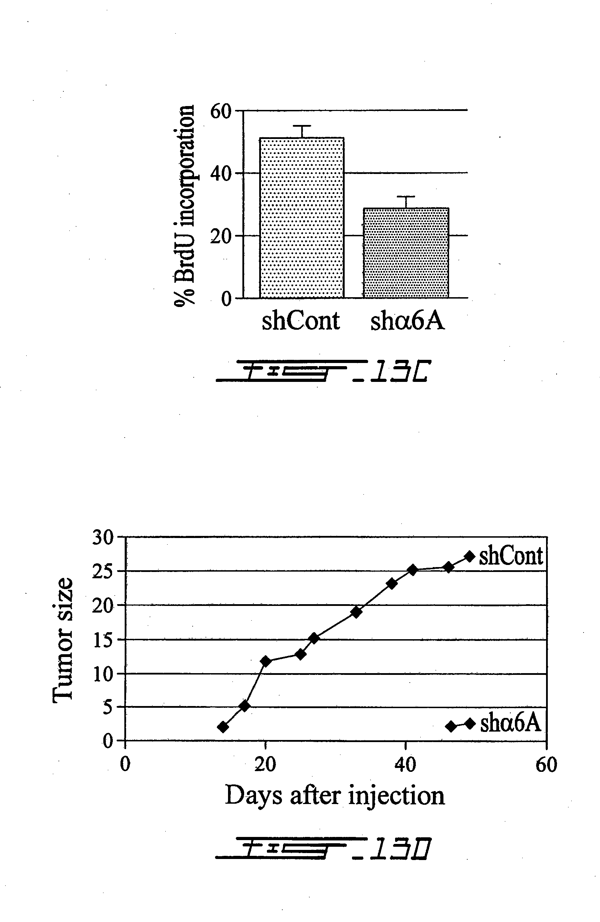

**Figure 13****.** Inhibition of the α6A isoform in Caco-2/15 limits their proliferation and their tumor-formation properties. (A) RT-PCR results of Shc (control) cells and Shα6A cells (shRNA treated-cells) showing the lack of expression of the α6A isoform in Shα6A cells. (B) Luciferase activity (Luc/Ren) for measuring TCF/β-catenin activity in Shc and Shα6A cells showing a decreased TCF/β-catenin activity in Shα6A cells. *: statistically significantly different from Shc cells. (C) Proliferation rate of the Shc and Shα6A cells showing a reduced proliferation of Shα6A cells. (D) Tumor growth (size in mm² in function of time (days)) of Shc and Shα6A cells injected into immuno-comprised mice indicating that Shα6A-derived tumor growth more slowly.

### DETAILED DESCRIPTION OF THE PREFERRED EMBODIMENT

In accordance with the present invention, it has been shown that the ratio between the B and A isoforms of the integrin α6 subunit is tied to cellular proliferation. This ratio is elevated in quiescent (e.g. differentiated) cells and low in proliferative (e.g. malignant) cells and thus can be used for the evaluation of cellular proliferation. This ratio can also be modulated to decrease or increase cellular proliferation.

The ITGA6 protein product is the integrin alpha chain alpha 6. Alpha 6 integrin subunit may combine with beta 4 in the integrin referred to as TSP180, or with beta 1 in the integrin VLA-6. It is mostly expressed in epithelial cells. Two transcript variants encoding different isoforms have been found for this gene: the B isoform (e.g. coding sequence accession number NM_000210 or SEQ ID NO: 1; polypeptide sequence accession number NP_000201 or SEQ ID NO: 2) and the A isoform (e.g. coding sequence accession number NM_001079818 or SEQ ID NO: 3; polypeptide sequence accession number NP_001073286 or SEQ ID NO: 4). Even though the ITGA6 protein has been shown to be upregulated in cancer, the present application provides suprising evidence that the ratio of the B and A isoforms is also modulated during cellular proliferation and can be modified to induce cellular proliferation or cellular quiescence. The present application also shows that the ratio between the two isoforms is an indicator of cellular proliferation and could successfully be used in the diagnosis of cancer or any other hypeproliferative state.

Various conditions are associated with either an hyperproliferative (such as cancer, psoriasis) or an hypoproliferative state of a cell. As indicated above, since the ratio between the B and A isoforms of the α6 integrin subunit is tightly linked to the ability of a cell to proliferate or enter into quiescence, the ratio can be successfully used to treat the above-noted conditions. Since the α6 integrin subunit is mostly expressed in epithelial cells, it is contemplated that the modulation of the B:A ratio in those cells will be particularly useful.

The present application describes a method of limiting the proliferation of a cell. As used herein, the term "inhibiting" refers to the ability of the method to lower or slow down the proliferation of a cell. In certain embodiments, the method is also capable of halting the proliferation of a cell by allowing the cell to exit the cellular cycle (e.g. G1 exit). As used herein, the term "proliferation" refers to the ability of a cell to complete a cell cycle. The proliferation of a cell can be assessed by determining the proliferation rate of a cell, e.g. the number of cell cycles completed by a cell in a definite amount of time (e.g. hours, days, weeks). Since the proliferation rate is not uniform between every cell type, care should be taken to determine if an "inhibition of the proliferation of a cell" has occurred by comparing the proliferation rate of the cell before and after the treatment or to a similar cell that has not been treated.

In order to inhibit the proliferation of a cell, the method comprises increasing the ratio B:A ratio of the α6 integrin in the cell. This is achieved by increasing the net amount of the B isoform of the integrin α6 subunit in the cell. In another embodiment, this can also be achieved by overexpressing the mRNA encoding the B isoform and/or increasing the stability of the mRNA encoding the B isoform in a cell. It can also be done by increasing the activity of the B isoform. It can further be achieved by reducing the amounts of the A isoform.

The modulation of expression of the B and A isoforms can be achieved using genetic engineering means. Such genetic means can be an vector appropriate for gene therapy that, when introduced in a cell, favors the expression of one isoform and consequently increases the B:A ratio balance of the α6 integrins in the cell. Another genetic means can be a vector appropriate for gene therapy that, when introduced in a cell, limits the expression of one isoform and consequently increases the B:A ratio balance of the α6 integrins in the cell (shRNA methodology for example). A combination of both types of vectors is also contemplated herein.

The modulation of expression of the B and A isoforms can be also be achieved using a small molecule (e.g. agent) that is capable of favoring or inhibiting the expression of one of the two (or both) isoforms or the activity of one (or both) isoforms.

As shown below, the increase of the B:A ratio of the isoform of integrin α6 subunit can also lead to the down-regulation of the activity of c-Myc in the treated cell. The c-Myc oncogene is a transcription factor that is upregulated in various hyperproliferative state, such as cancer. As such, the methods presented herein can advantageously be used in cells (or conditions related thereto) overexpressing c-Myc or having an enhanced c-Myc activity (with respect to a control healthy cell or control healthy tissue) to lower c-Myc expression and/or activity.

The method described herein can be preferably applied to cells (from human or animal origin) that express (either endogenously or recombinantly) the A and B isoforms of the integrin α6 subunit. As indicated above, some epithelial cells endogenously express both isoform and can be advantageously used in this method.

Epithelial cells compose the epithelium, a tissue that lines the cavities and surfaces of structures throughout the body. Epithelial cells usually lie on top of connective tissue, and the two layers are separated and linked by a basement membrane. Epithelial tissue can be divided into two groups depending on the number of layers of which it is composes. Epithelial tissue which is only one cell thick is known as simple epithelium. If it is two or more cells thick, it is known as stratified epithelium. Simple epithelium can be subdivided according to the shape and function of its cells:
- Squamous (pavement) epithelium. Squamous cells have the appearance of thin, flat plates. Squamous cells, for example, tend to have horizontally flattened, elliptical nuclei because of the thin flattened form of the cell. They form the lining of cavities such as the mouth, blood vessels, heart and lungs and make up the outer layers of the skin.
- Cuboidal epithelium. As their name implies, cuboidal cells are roughly square or cuboidal in shape. Each cell has a spherical nucleus in the centre. Cuboidal epithelium is found in glands and in the lining of the kidney tubules as well as in the ducts of the glands. They also constitute the germinal epithelium which produces the egg cells in the female ovary and the sperm cells in the male testes.

- Simple columnar epithelium. Columnar epithelial cells occur in one or more layers. The cells are elongated and column-shaped. The nuclei are elongated and are usually located near the base of the cells. Columnar epithelium forms the lining of the stomach and intestines. Some columnar cells are specialised for sensory reception such as in the nose, ears and the taste buds of the tongue. Goblet cells (unicellular glands) are found between the columnar epithelial cells of the duodenum. They secrete mucus or slime, a lubricating substance which keeps the surface smooth.
- Ciliated columnar epithelium. These are simple columnar epithelial cells, but in addition, they possess fine hair-like outgrowths or cilia on their free surfaces. These cilia are capable of rapid, rhythmic, wavelike beatings in a certain direction. This movement of the cilia in a certain direction causes the mucus, which is secreted by the goblet cells, to move (flow or stream) in that direction. Ciliated epithelium is usually found in the air passages like the nose. It is also found in the uterus and Fallopian tubes of females.
- Glandular Epithelium. Columnar epithelium with exocrine cells is called glandular epithelium. Some parts of the glandular epithelium consist of such a large number of exocrine cells that there are only a few normal epithelial cells left. Columnar and cuboidal epithelial cells often become specialised as gland cells which are capable of synthesising and secreting certain substances such as enzymes, hormones, milk, mucus, sweat, wax and saliva. Unicellular glands consist of single, isolated glandular cells such as the goblet cells. Sometimes a portion of the epithelial tissue becomes invaginated and a multicellular gland is formed. Multicellular glands are composed of clusters of cells. Most glands are multicellular including the the salivary glands.
- Stratified Epithelium. Where body linings have to withstand wear and tear, the epithelia are composed of several layers of cells and are then called compound or stratified epithelium. The top cells are flat and scaly and it may or may not be keratinised. The mammalian skin is an example of dry, keratinised, stratified epithelium. The lining of the mouth cavity is an example of an unkeratinisied, stratified epithelium.

Epithelial cells can be derived, for example, from blood vessels, gingiva, tongue, digestive ducts of submandibular glands, gallbladder, hard palate, large intestine, oesophagus, rectum, small intestine, stomach, thyroid follicles, mesothelium of body cavities, skin ,sweat gland ducts, lymph vessel, ependyma, cervix (ectocervix and endocervix), endometrium, Fallopian tubes, labia majora, ovaries, uterus, vagina, ductuli efferentes, ejaculatory duct, epididymis, rete testis, tubuli recti, vas deferens, seminal vesicle, larynx, oropharynx, bronchioles, trachea, respiratory epithelium, cornea, olfactory epithelium, urethral orifice, kidney, prostatic urethra, renal pelvis, ureter, urinary bladder, etc.

The methods described herein can be applied to cells derived from the gastro-intestinal tract such as, for example, those of the colon. Even though the methods described herein can be applied to any type of cells, because a low B:A ratio is indicative of an hyperproliferative state, the treatment method described herein can be advantageously used for the inhibition of proliferation of a malignant cell. The malignant cell can be from either a primary tumor or a metastasis.

As indicated above and shown below, a low B:A ratio of the α6 integrin is associated with an hyperproliferative state. As such, in cells where proliferation, rate should be increased to return or achieve to homoestasis, the present application also provides a method of increasing the proliferation of a cell. This method comprises decreasing the ratio B:A ratio of the α6 integrin. This is achieved by expressing preferably the A isoform of the integrin α6 subunit in the cell. This can also be achieved by increasing the stability of the mRNA encoding the A of the integrin α6 subunit in a cell.

The modulation of expression of the B and A isoforms can be achieved using genetic engineering means. Such genetic means can be an vector appropriate for gene therapy that, when introduced in a cell, favors the expression of one isoform and consequently decreases the B:A ratio balance of the α6 integrins in the cell. Another genetic means can be a vector appropriate for gene therapy that, when introduced in a cell, limits the expression of one isoform and consequently decreases the B:A ratio balance of the α6 integrins in the cell. A combination of both types of vectors is also contemplated herein.

The modulation of expression of the B and A isoforms can be also be achieved using a small molecule (e.g. agent) that is capable of favoring or inhibiting the expression of one of the two (or both) isoforms or the activity of one (or both) isoforms.

Cells (animal or human) having a specific need for augmenting their proliferation rate can be submitted to the method described herein. Care should be taken in applying this methods not to dysregulate cellular proliferation and cause a cancer or augment the predisposition of cancer to the treated cells. It is also contemplated that the method described herein may favor the up-regulation of the activity of c-Myc in the treated cells.

In order to augment the proliferation of a cell, the method contemplates either overstranscribing or increasing the stability an mRNA encoding the isoform A so as to increase the expression of the isoform A of the integrin α6 subunit. The mRNA is encoded by a recombinant nucleic acid that can, optionally, be introduced into the cell. In another embodiment, the method also contemplates increasing the activity or stability of the isoform A so as to increase the expression of the isoform A (endogenous or recombinantly introduced) of the integrin α6 subunit.

Also described herein is the use of an agent capable of increasing the expression or transcription of the B isoform for the inhibition of proliferation as well as for the manufacture of a medicament for the inhibition of proliferation of a cell. Such agent augments the ratio between the B and A isoforms. In addition, the use of an agent capable of increasing the expression or transcription of the A isoform for the promotion of proliferation as well as for the manufacture of a medicament for the promotion of proliferation of a cell is also described. In that case the agent preferably lowers the ratio between the B and A isoforms.

Since the B:A ration of the isoforms of the integrin α6 subunit is linked to the proliferation state of a cell, it can also be successfully used to as an indicator of cellular proliferation (e.g. proliferative state vs. quiescence). In return, this indicator can be used to determine the risk associated with the onset of an hyperproliferative cellular condition (such as cancer).

The invention relates to a method of diagnosing an hyperpoliferative state in an individual is contemplated herewith. In a first step, the method comprises determining a ratio between the isoform B and the isoform A of an integrin α6 subunit in a cell (such as an epithelial cell) from the individual. This determination can either be made at the genomic level, transcript level and/or at the polypeptide level. In an embodiment, the determination can be made at a first level (transcript of polypeptide) and confirmed at a second level (transcript or polypeptide).

This diagnostic method can be embodied in a diagnostic system designed to perform the required steps. This diagnostic system comprises at least two modules: a first module for performing the determination of the ratio between the α6B and α6A isoforms and a second module for correlating the ratio to an hyperproliferative or an hypoproliferative state. The first module can comprise a detection module for determining the amount of α6B and α6A isoforms as well as a processor to calculate the ratio between the isoforms. As indicated above, this detection can be made either at the RNA level and/or the polypeptide level. The detection module relies on the addition of a label to the sample and the quantification of the signal from the label for determining the amount of the α6B and α6A isoforms. The signal of the label is quantified by the detection module and is linked to the amount of the α6B and α6A isoforms. This label can directly or indirectly be linked to a quantifier specific for the two isoforms. The detection module then processes the amounts obtained to generate a ratio of the two isoforms. The information (e.g. amount and ratio) gathered by the detection module is then processed by the second module for determining the correlation. This second module can use a processor for comparing the ratio obtained with the first module to a reference or control ratio (predetermined or obtained from an individual (or group of individuals) that are not afflicted with a proliferative disease and are thus considered healthy). The correlation module can then determine if the ratio obtained from the determination module is more likely associated with hyperproliferation, hypoproliferation or homeostasis and as such, the individual's susceptibility of having or developing the disease associated with proliferation.

As indicated above, the determination of the ratio can include the addition of a quantifier to the sample from the individual. The quantifier is a physical entity that enables the sample to be quantified. The sample can be purified or isolated prior to the addition of the quantifier. The quantifier can be, for example, an oligonucleotide specific for the isoform to be quantified, an antibody specific for the polypeptide to be quantified or a ligand specific for the enzyme to be quantified. The addition of the quantifier generates a quantifiable sample that can then be submitted to an assay for the determination of the quantity of nucleic acid and/or polypeptide. The quantifier is either directly linked to a label or adapted to be indirectly linked to a label for its processing in the detection module.

Once the concentration or amount of both isoforms has been determined, a ratio between the B and A isoform can be calculated and compared to a control ratio between the B and A isoform. A control ratio between the B and A isoform of the α6 integrin is either a value of about 1.5 (e.g. between 1.3 and 1.7) or the B:A ratio that has been determined in a cell type similar to the one of the cells of the sample and that is considered to have a normal proliferation rate. For example, a control ratio of epithelial cells can be calculated by first determining the amount or concentration of the B and A isoforms of the α6 integrin in a sample of a cell derived from a healthy individual known not be afflicted with a hyperproliferative state such as cancer (e.g. an individual free of cancer). Optionally or alternatively, the control ratio could also be calculated in cells/tissues from an individual experiencing cancer but derived from a healthy section of a tissue which is not associated with an hyperpoliferative disease (such as a resection margin). A B:A ratio associated with an hyperproliferative disease, especially for colon cancer cells, is lower than 1.5 and is usually of about 1.1 (e.g. between 0.9 and 1.3).

This diagnostic method is particularly useful in the determination of cancer and, in an embodiment, carcinoma (cancer associated with epithelial cells). The diagnostic method described herein is not limited to any type of cancer. However, it can be successfully used in the diagnosis of cancers associated with the gastro-intestinal tract, such as colon cancer.

The present invention will be more readily understood by referring to the following examples which are given to illustrate the invention rather than to limit its scope.

### EXAMPLE I - Expression of the α6 variants in the human intestine

*Tissues.* Primary tissues of healthy adult ileum were obtained from Quebec Transplant (Quebec, Canada). Primary extracts of fully differentiated villus enterocytes were obtained according to a previously published protocol (Perreault and Beaulieu, 1998). All tissues were obtained in accordance with protocols approved by the local Institutional Human Research Review Committee. The preparation and embedding of tissues for cryosectioning and RNA extraction was performed as described previously (Ni et al., 2005).

*Primary antibodies.* An antibody recognizing both splice variants of integrin α6 (G0H3) (Sonnenberg et al., 1987) and antibodies recognizing α6A (1A10) and α6B (6B4) (Hogervorst et al., 1993) were obtained from Dr. A. Sonnenberg (Division of Cell Biology, The Netherlands Cancer Institute, Amsterdam). Subsequently, these antibodies were obtained from Santa Cruz™ (Santa Cruz, CA; G0H3), Chemicon™ (Temecula, CA; 1A10) and MUbio Products™ (Maastricht, The Netherlands; 6B4). A rabbit polyclonal α6A (α6-cytoA) (de Curtis and Reichardt, 1993) was obtained from Dr. de Curtis (Department of Molecular Pathology and Medicine, San Raffaele Scientific Institute, Milan, Italy), anti-Ki67 (KiS5, Chemicon™), anti-lysozyme (DAKOCytomation™) and anti β-actin (C4, Chemicon™) were also used.

*Indirect immunofluorescence.* Cryosections were fixed in 2% paraformaldehyde for the detection of α6, α6A, Ki67 and lysozyme or in -20°C ethanol for the detection of α6B and processed as described previously (Ni et al., 2005). In all cases, no immunofluorescent staining was observed when a mix of mouse and rabbit non-immune sera replaced primary antibodies.

*Western blot.* Western blots were performed as SDS-PAGE under non-denaturing conditions as previously described (Ni et al., 2005). After transfer of the separated samples to a nitrocellulose membrane (BioRad™, Hercules, CA) unspecific protein binding to the membrane was blocked by 2% BSA/0.1% Tween™ followed by incubation with the α6A 1A10 monoclonal antibody. Following detection, the membrane was stripped of antibody by incubation in stripping solution (50mM Tris (pH 6.8), 2% SDS, 100mM β-mercaptoethanol) at 50°C for 20 minutes after which the membrane was reprobed with the α6B 6B4 antibody using 2% BSA/0.1% Tween™ as blocking solution. Finally, the membrane was restripped and reprobed with a β-actin antibody in 5% skim milk powder/0.1 % Tween™ as an input control.

*Plasmids and plasmid construction.* The β-catenin/TCF4 responsive luciferase reporter plasmid, TOPFlash™ (Upstate, Charlottesville, VA) has been characterized elsewhere (Korinek et al., 1997). Firefly luciferase reporter plasmids carrying promoters of the differentiation markers lactase-phlorizin hydrolase (pGL3-LPH1085-13910T) (Troelsen et al., 2003), intestinal alkaline phosphatase (pALPI_566) (Olsen et al., 2005) and sucrase-isomaltase (pSI-202/+54) (Boudreau et al., 2002) have been characterized elsewhere. The dipeptidyl peptidase IV (DPPIV) promoter plasmid was generated in our lab by PCR-amplification of 1382 bp of the immediate 5' promoter of DPPIV (sense primer: 5'- CGGGGTACCTTGGAAGAGGGAGGAGGAG -3' (SEQ ID NO: 5), antisense primer: 5'- GAAGATCTAGTCACTCGCCGCTGGCA -3' (SEQ ID NO: 6)) followed by *Kpn I* and *Bgl II* (underlined sequences) mediated insertion into pGL3, yielding the plasmid pGL3/Prom.dppIV. An expression vector containing the cDNA of integrin α6A, pRc/CMV-α6A (Delwel et al., 1993), was obtained from Dr. Sonnenberg (Division of Cell Biology, The Netherlands Cancer Institute, Amsterdam, The Netherlands). For α6B, the cDNA encoding the cytoplasmic tail of the integrin α6A subunit in pRc/CMV-α6A was replaced by the cDNA encoding the cytoplasmic tail of the integrin α6B subunit by *XbaI* digestion of the recipient (pRc/CMV-α6A) and donor (pPCR-Script-α6B) vectors followed by ligation, generating pRc/CMV-α6B.

*Cell culture.* The crypt-like human intestinal epithelial HIEC cells and Caco-2/15 cells were grown as described previously (Basora et al., 1999; Perreault and Beaulieu, 1996; Vachon and Beaulieu, 1995).

*RT-PCR.* Primers used to co-amplify the α6A and α6B transcripts were sense: 5'-CTAACGGAGTCTCACAACTC-3' (SEQ ID NO: 7) and antisense: 5'-AGTTAAAACTGTAGGTTCG -3' (SEQ ID NO: 8). Each cycle was composed of template denaturation at 94°C for 1 minute, primer annealing at 65°C for 1 minute and elongation at 72°C for 1 minute. The primer annealing temperature was decreased by 0.5°C after each round of amplification for 40 cycles followed by a final 15 cycles at an annealing temperature of 45°C.

*Transfection and luciferase measurement.* Caco-2/15 cells were transfected using FuGENE™ transfection agent (Roche, Indianapolis, IN). Firefly and renilla luciferase activities were measured using the Dual-Lucifierase® Reporter Assay System (Promega Corporation, Madison, WI) according to the manufacturer's instructions as described previously (Escaffit et al., 2005).

As shown previously for the β4 subunit (Basora et al., 1999), immunodetection of the α6 integrin subunit using an antibody directed against the extracellular domain (G0H3) (Sonnenberg et al., 1987) yielded ubiquitous staining at the base of the epithelial cells in both villus and crypt (Fig. 1A). Staining for the integrin α6A subunit was observed in the epithelium and was found to be restricted to proliferative cells in the lower-middle to upper crypts with a fade out of staining at the base of the villus (Fig. 1B, Fig. 2A). Additional labeling was seen in the vasculature of the lamina propria. In contrast, staining for the α6B variant was found to be predominant at the base of villus epithelial cells and at the bottom of the crypts, while relatively weak staining was detected in the middle to upper regions of the crypts (Fig. 1C, Fig. 2B). The relation of α6A and α6B expression to intestinal cell proliferation and differentiation, respectively, was confirmed by double staining with specific proliferation and Paneth cell markers. As shown in Figure 3, expression of the α6A subunit was found to be above the Paneth cell region as determined with lysozyme co-immunostaining (Fig. 3A) and adjacent to the rich Ki67-positive region as determined in the corresponding crypt from serial cryosections (Fig. 3B). In contrast, expression of the α6B subunit was predominantly detected in the upper crypt/lower villus region and co-localized at the bottom of the crypts with differentiated Paneth cells as identified by lysozyme (Fig. 3C). This pattern of expression was consistently observed and the images shown are representative of the 6 samples studied.

A competitive RT-PCR was then performed using primers that amplify the transcripts of both α6 variants from cDNA originating from the normal crypt-like human cell line HIEC and primary human villus epithelial cells, as well as from the Caco-2/15 cell line that undergoes an intestinal differentiation program at postconfluence. A clear shift from a high α6A/α6B transcript ratio to a low ratio was seen accompanying differentiation at different stages of enterocytic differentiation (Fig. 4; Statistically significantly different from SC, p < 0.05, Tukey's One Way Analysis of Variance (ANOVA), n=3). A similar shift was observed in Caco-2/15 cells at the protein level (Fig 4B; Statistically significantly different from SC, p < 0.01, Tukey's One Way Analysis of Variance (ANOVA), n=3)). The findings that the two splice variants of the α6 integrin are differentially expressed in the proliferative and differentiated compartments of the gut, and that there is an association between the ratio of these two splice variants with the stage of differentiation, are consistent with previous findings of distinct expression of components of the integrin-ECM system in gastrointestinal biology (Basora et al., 1999; Basora et al., 1997) supporting the importance of interactions between the epithelium of the intestinal tract and the underlying BM (Beaulieu, 1997; Teller and Beaulieu, 2001).

Distinct patterns of expression for the two α6 variants have been reported in different organs during development (de Curtis and Reichardt, 1993; Segat et al., 2002; Thorsteinsdottir et al., 1995) suggesting a functional importance of the expression ratio of the two forms. The exclusive expression of α6A in a rapidly dividing cellular compartment is known from the epidermis (Hogervorst et al., 1993). Interestingly, it has been suggested that the ratio of the two variants can determine cellular behavior and that a proper cellular response is dependent on the presence of both variants rather than a substitution of one with the other (Segat et al., 2002). As shown herein, a modulation of the α6A/α6B ratio in intestinal cells was observed rather than the replacement of one α6 variant with the other.

In order to verify whether the reduction of the α6A/α6B ratio was related to differentiation, Caco-2/15 cells were co-transfected with reporter vectors carrying promoters of the enterocytic differentiation markers sucrase-isomaltase, intestinal alkaline phosphatase, lactase-phlorizin hydrolase or dipeptidyl peptidase IV (DPPIV) and expression vectors encoding α6A (pRc/CMV-α6A) or α6B (pRc/CMV-α6B). As illustrated with DPPIV (Fig. 5), independent experiments revealed no differential activation of any of the four tested promoters in newly confluent Caco-2/15 cells. Without wishing to be bound to theory, these results suggest that α6Bβ4 is not involved in the initiation of differentiation although a role in modulating differentiation at later stages or regulating other functions such as cell migration cannot be excluded. Conversely, modulation of the early steps of intestinal cell differentiation observed in response to cell-laminin interactions (Vachon and Beaulieu, 1995) could be mediated by other laminin receptors such as the α7Bβ1 integrin (Basora et al., 1997).

The ability of the two variants to differentially affect intracellular pathways associated with enterocytic proliferation was performed by co-transfecting the two integrin α6 variants with a reporter plasmid responding to β-catenin/TCF (TOPFlash™) activity. This activity is associated with cell-cycle progression (Korinek et al., 1997). The β-catenin/TCF complex was found to be significantly and specifically stimulated by α6A (Fig. 6) suggesting a link between α6A expression and promotion of cell proliferation. However, stable expression of the α6A subunit in Caco-2/15 cells did not result in a net increase in cell proliferation (Dydensborg et al., unpublished data) suggesting that the β-catenin pathway linked to the regulation of cell proliferation in this APC-mutated colon cancer cell line (Ilyas et al., 1997) is already at maximal stimulation.

It was previously demonstrated that the α6β4 integrin is the only α6 containing integrin in the human intestine (Basora et al., 1999). In this context, it is noteworthy that there is substantial evidence for the differential capacity of the α6Aβ1 and α6Bβ1 integrins to initiate intracellular signaling (Shaw et al., 1995; Wei et al., 1998) and facilitate migration on laminin (Shaw and Mercurio, 1995). However, no study has ever demonstrated a functional difference between the α6Aβ4 and α6Bβ4 integrins. The finding that the α6Aβ4 integrin is predominant in intestinal proliferative cells both in the intact intestine and in established intestinal cell lines suggests that the α6A/α6B ratio plays an important role in intestinal homeostasis.

### EXAMPLE II - Integrin α6β4 and colon cancer cell proliferation,

*Tissues.* Samples of adult colon were obtained from patients between the ages of 49 and 86 years who had undergone surgical treatment for colon adenocarcinoma. For each patient, samples from the primary tumor and from non-diseased areas (at least 10 cm distant from the lesion) corresponding to the resection margin were obtained. Diagnoses of adenocarcinoma were confirmed by pathologists. Staging of the carcinomas was according to Astler and Coller (1954). Resection margins of colon specimens obtained from patients undergoing surgery for pathologies other than colon cancer (bowel obstructions, diverticulosis, etc.) were also used for immunofluorescence. All tissues were obtained in accordance with protocols approved by the local Institutional Human Research Review Committee for the use of human material. The preparation and embedding of tissues for cryosectioning and RNA extraction was performed as described previously (Beaulieu, 1992). Additional paired samples (resection margin and confirmed carcinomas) were obtained from the Cooperative Human Tissue Network (Midwestern Division, Ohio State University, OH, USA), which is funded by the National Cancer Institute.

*Indirect immunofluorescence.* Cryosections 3 µm thick were fixed in 2% paraformaldehyde (α6A, Ki67 and Rbm19) or -20°C ethanol (α6B). Nonspecific protein-protein interactions were blocked for one hour at room temperature by immersion of slides in 10% goat serum (α6A) or 2% BSA (α6B) in PBS followed by incubation with the primary antibodies diluted 1:200 in their respective blocking solutions overnight at room temperature. Following extensive washing in PBS, the slides were incubated with either FITC or rhodamine conjugated secondary antibodies raised against mouse and rabbit IgG (Chemicon™), respectively, for one hour at room temperature before being washed in PBS. The slides were stained with Evans Blue (0.01% in PBS) before being mounted in glycerol:PBS (9:1) containing 0.1% paraphenylenediamine and observed for fluorescence with a Leica Reichart Polyvar 2™ microscope (Leica Canada, Saint-Laurent, QC) equipped with a Leica DFC300 FX ™ digital color camera. In all cases, no immunofluorescent staining was observed when a mix of mouse and rabbit non-immune sera replaced primary antibodies.

*Primary antibodies.* Two antibodies recognizing integrin α6A (1A10) and α6B (6B4) (Hogervost et al., 1993) were obtained from Dr. A. Sonnenberg (Division of Cell Biology, The Netherlands Cancer Institute, Amsterdam, The Netherlands). Subsequently, these antibodies were obtained from Chemicon™ (Temecula, CA; 1A10) and MUbio Products™ (Maastricht, The Netherlands; 6B4). Mabs 1A10 and 6B4 were used for western blots and co-immunoprecipitation. For indirect immunofluorescence, 6B4 and a rabbit polyclonal α6A (α6-cytoA) (de Curtis and Reichardt, 1993) antibody was obtained and employed in place of 1A10. This antibody was obtained from Dr. de Curtis (Department of Molecular Pathology and Medicine, San Raffaele Scientific Institute, Milan, Italy). The anti-Ki67 monoclonal antibody KiS5 and the polyclonal anti-lysozyme antiserum were from Chemicon™ and DAKOCytomation™ (Glostrup, Denmark). The anti-progenitor cells Rbm19 antibody (Lorenzen et al., 2005) was obtained from Dr. Alan M. Mayer (Department of Pediatrics, Medical College of Wisconsin, WI). Bin1 was detected using 99D from Santa Cruz™ (Santa Cruz, CA). To probe for β-actin, the antibody C4 from Chemicon™ was employed.

*Plasmids and plasmid construction.* The c-Myc responsive luciferase reporter plasmid, pMyc-TA-Luc™ (Clontech, Mountain View, CA), carrys six c-Myc binding sequences in front of the minimal TATA box from the herpes simplex thymidine kinase (HSV-TK) promoter. An Rb responsive luciferase reported plasmid, pRb-TA-Luc™ (Clontech) was also used. An expression vector containing the cDNA of integrin α6A, pRc/CMV-α6A (Delwel et al., 1993), was obtained from Dr. Sonnenberg (Division of Cell Biology, The Netherlands Cancer Institute, Amsterdam, The Netherlands). The α6A cDNA was subcloned into the viral expression vector pLPCX™ (BD Bioscience Clontech, Mississauga, ON) by a non-directional strategy using *HindIII.* Correct orientation was verified by restriction enzyme analysis. cDNA originating from a preparation of fetal epithelial enterocytes separated from the mesenchyme using Matrisperse™ (BD Biosciences, Mississauga, ON) as described previously (Perreault and Beaulieu, 1998) was used as a template for PCR amplification of the cytoplasmic tail of the α6B subunit using Pwo polymerase™ (Roche, Laval, QC). The upstream primer (5' TGCTGAAAGAAAATACCAGA 3' (SEQ ID NO: 9)) spanned an endogenous *XbaI* site, while the downstream primer (5' GCTCTAGAGAAAAAGCAGTTTGGGTACT 3' (SEQ ID NO: 10)) introduced another (underlined sequence). The amplified DNA was ligated into pPCR-Script™ (Stratagene, La Jolla, CA) and verified for fidelity by sequencing. Subsequently, the cDNA encoding the cytoplasmic tail of the integrin α6A subunit in pRc/CMV-α6A was replaced by the cDNA encoding the cytoplasmic tail of the integrin α6B subunit by *XbaI* digestion of the recipient (pRc/CMV-α6A) and donor (pPCR-Script-α6B) vectors followed by ligation, generating pRc/CMV-α6B. The cDNA encoding the integrin α6B subunit was subcloned into the pLPCX vector using the same strategy as for α6A. A mammalian episomal expression vector, pEEP1™, was used to generate populations of Caco-2/15 cells over-expressing the two integrin α6 splice variants. Integrin α6A or α6B subunit cDNA was excised from pRc/CMV-α6A and pRc/CMV-α6B, respectively, using *HindIII,* Klenow filled and blunt-end ligated into a Klenow filled *NotI* site in pEEP1, generating pEEP1-α6A and pEEP1-α6B.

*Cell culture and generation of colon cancer cells over-expressing α6A and α6B.* The colon cancer cell line, Caco-2/15 was grown in DMEM™ (GIBCO, Burlington, ON) supplemented with 10% fetal bovine serum (ICN Biomedicals, Aurora, OH), 1% HEPES and 1% Glutamax™ (both from GIBCO, Burlington, ON) as described previously (Beaulieu and Quaroni, 1991). The colon cancer cell lines HT-29, COLO 201, DLD-1, HCT 116, T84, SW480 and SW620 were grown in accordance with instructions provided by the ATCC (Rockville, MD). All cells were grown in an atmosphere of 95% air and 5% CO₂ at 37°C.

The pEEP1-α6A and pEEP1-α6B plasmids were introduced into 1x10⁶ Caco-2/15 cells by nucleofection using the Amaxa Biosystem Nucleofection kit™ (ESBE Scientific, St.-Laurent, QC) using the T-20 setting. Immediately following nucleofection, the cells were seeded onto collagen coated cell culture dishes (Falcon, Franklin Lakes, NJ). 24 hours post-nucleofection the cells were subjected to hygromycin (Multicell, St.-Bruno, QC) selection at a concentration of 200 µg/ml. This selection pressure was maintained throughout the experimental period to ensure continuous replication and transfer of the episomal plasmid. Forced expression of the α6A and α6B subunits was monitored by western blot.

*BrdU incorporation and staining.* BrdU incorporation and staining was performed according to the manufacturer's (Roche Applied Science, Laval, QC) instructions. Briefly, 24 hours after plating of cells in LabTeks™ (Nalgene Nunc, Rochester, NY) the cells were incubated for two hours with normal medium containing BrdU then immediately subjected to BrdU and DAPI staining. For each condition, two random fields per well were counted for DAPI and BrdU positive cells and the BrdU positive cell population was determined as a percentage of total DAPI positive cells. All experiments were performed in quadruplicate and repeated three times.

*Western blot and immunoprecipitation.* Western blots were performed as SDS-PAGE under non-denaturing conditions using 120 µg of whole cell lysate per lane. After transfer of the separated samples to a nitrocellulose membrane (BioRad, Hercules, CA) unspecific protein binding to the membrane was blocked by 5% skim milk-powder in Pubs-0.1% Tween™ followed by incubation with the α6A 1A10 monoclonal antibody. Following detection, the membrane was stripped of antibody by incubation in stripping solution (50mM Tris (pH 6.8), 2% SDS, 100mM β-mercaptoethanol) at 50°C for 20 minutes after which the membrane was reprobed with the α6B 6B4 antibody using 2% BSA/0.1% Tween™ as blocking solution. Finally, the membrane was restripped and reprobed with an β-actin antibody as an input control.

For immunoprecipitation of α6β4 and α6β1, newly confluent Caco-2/15 cells and keratinocytes (obtained from Dr L. Germain, LOEX, Universite Laval, Quebec, QC) were metabolically labeled using Promix™^{[35S]}methionine and cystine (Amersham Pharmacia Biotech), 200 µCi/ml for 6 h. Cells were lysed and processed as previously described (Basora et al., 1999) for immunoprecipitation of α6-containing integrins with the antibody G0H3 and Protein-G Sepharose™ (Invitrogen). Radioactive samples were analyzed under reduced and nonreduced conditions by SDS PAGE (Basora et al., 1999).

*RT-PCR.* First strand cDNA synthesis was performed with 2 µg total RNA using oligo(dT)₁₂₋₁₈™ (Amersham Pharmacia, Bay d'Urfé, QC) as primer and Omniscript reverse transcriptase™ (Qiagen, Mississauga, ON) for synthesis. Primers used to co-amplify the α8A and α6B transcripts using 1/50 of the synthesized cDNA above were sense: 5'-CTAACGGAGTCTCACAACTC-3' (SEQ ID NO: 7) and antisense: 5'-AGTTAAAACTGTAGGTTCG -3' (SEQ ID NO: 8). Each cycle was composed of template denaturation at 94°C for 1 minute, primer annealing at 65°C for 1 minute and elongation at 72°C for 1 minute. The primer annealing temperature was decreased by 0.5°C after each round of amplification for 40 cycles followed by a final 15 cycles at an annealing temperature of 45°C.

*Real-time quantitative RT-PCR.* Quantitative RT-PCR was performed as previously described (Dydensborg et al., 2006). Three different primer pairs for the integrin α6 subunit were tested for amplification efficiency and fidelity. The primer pair termed α6PD-2 was chosen for amplification of cDNA coding for the integrin α6 subunit based on a superior amplification efficiency and lack of primer dimer formation as assessed by melting curve analysis. The Cₜ-values were converted into relative expression values compared to a pooled RNA standard (QPCR Human Reference Total RNA™, Stratagene, La Jolla, CA) before normalization of α6 expression against a weighted average of three normalizing genes (B2M, MTR & MAN1B1) using the geNorm applet (Vandesompele et al., 2002). Briefly, this algorithm normalizes a gene of interest against several normalizing genes, rather than against a single gene, thus obtaining an analysis of expression that is less likely to be impacted by any random fluctuations in the expression level of the normalizing gene(s). The sequences of the α6PD-2 primer pairs were: sense 5' TGGGATATGCCTCCAGGTT 3' (SEQ ID NO: 11), antisense 5' TGTAGCCACAGGGTTTCCTC 3' (SEQ ID NO: 12). Primer pairs for B2M, MAN1B1, and MTR have been described previously (Dydensborg et al., 2006). The annealing temperatures of the reactions were 57°C (α6) or 58°C (B2M, MAN1B1 and MTR) and the amplification efficiencies of the reactions were 100.7%, 105.7%, 98.9% and 96.8% for α6, B2M, MTR and MAN1B1, respectively, as determined by standard curve analysis.

*Transfection and luciferase measurement.* Caco-2/15 cells were seeded in 24-well plates (Falcon, Franklin Lakes, NJ) and grown to 40-60% confluence before being transiently transfected in serum-free medium using FuGENE™ transfection agent (Roche, Indianapolis, IN) in a µg DNA to µl transfection agent ratio of 1:9. Cells were kept under normal growth conditions after transfection. All transfections were performed as co-transfections using a renilla luciferase expression plasmid to establish an internal control for transfection efficiency. Promoter activities of the various reporter plasmids were expressed using the arbitrary unit "RLU" (relative luciferase units). Numeric values of CMV promoters in control transfections (empty vector) were kept equal to experimental (α6A and α6B) numeric values by adjusting the absolute level of plasmids as measured by µg. DNA concentrations in transfections were kept constant with the addition of pBluescript SK+™ (Stratagene, Cedar Creek, TX). Equal amounts (25 ng) of reporter plasmid and expression vector (pRc/CMV-αE6A and pRc/CMV-α6B) were cotransfected with 2 ng of pCMV-Renilla per well. Firefly and renilla luciferase activity was measured using the Dual-Luciferase® Reporter Assay System (Promega Corporation, Madison, WI) according to the manufacturer's instructions using an Orion microplate luminometer™ from Berthold (Montreal Biotech, Kirkland, QC) for detection of the chemiluminescent signal. Individual experimental results were normalized to the average of the RLU of the empty vector cotransfectant in the corresponding experiment.

*Expression of α6 subunit variants in the normal colon.* The ubiquitous presence of the α6 dimerization partner β4 along the glandular unit of the human colon has already been shown (Ni et al., 2005). The delineation of the specific expression patterns of the α6A and α6B splice variants in the normal human adult colonic mucosa was thus performed (Fig. 7). The α6A subunit was expressed in the basal membrane of epithelial cells concentrated in the lower half of the glands (Fig. 7A), a region that contains the progenitor cells as identified by co-immunodetection with the proliferative antigens Ki67 and Rbm19 (Fig. 7B,C, G, H, I). Staining for the α6B subunit on serial sections revealed its presence in the epithelium to be located in the upper half of the glands and at the base of the surface epithelium (Fig. 7D, E), while it was not detected in the lower crypt (Fig. 7F). The association of α6A with the actively growing cell population and α6B with the mature differentiated population supports the hypothesis that each variant possesses a specific distinct function, necessary for intestinal homeostasis.

*a6 is up-regulated in colon cancer cells and undergoes a shift away from the α6B variant.* The α6β4 integrin is frequently up-regulated in several cancer types (Guo and Giancotti, 2004). Using quantitative PCR, it was observed that a significant up-regulation of total α6 expression in paired primary tumor samples versus patient matched normal resection margins (RM) (Fig. 8A) (Ni et al., 2005). It was then assessed whether modulation of α6 splice-variant expression accompanied the overall up-regulation of total α8 in primary tumors. Competitive RT-PCR using primers that amplify the transcripts of both α6 variants showed an overall dominance of α6B expression in the healthy resection margins (Figure 8B - RM). Furthermore, a clear down regulation of expression of the α6B variant in conjunction with a possible increase in the α6A variant were observed in patient matched tumors (Fig. 8B, C). Overall, 81% of the 21 pairs showed the same change in expression in the tumor sample, resulting in a statistically significant shift towards a diminished α6B/A-ratio in primary cancers compared to healthy resection margins (Fig. 8D). However, no correlation was noted between tumor grade or stage and α6B down regulation. A predominant expression of the α6B variant in normal colonic tissue is in accordance with the fact that the quiescent cell population outsizes the proliferative cell population in the normal colon. Cofrespondingly, a shift towards higher expression of the A variant in hyperproliferative colon carcinomas is in accordance with the association of this variant with the proliferative compartment of the healthy colon.

It was next investigated if the relative α6A and α6B levels in 6 well established colon cancer cell lines to see if this characteristic was conserved. All six cell lines tested predominantly expressed the A-variant (Fig. 9E). However, at least one exception to this exists since the Colo 320 cell line has been previously shown to predominantly express the B-variant (Hogervorst et al., 1993) suggesting that, as observed herein for the primary tumors where 4 out of the 21 pairs analyzed did not show an α6A subunit increase (Fig. 10D), the relation between high α6A expression and a pro-proliferative state is not absolute in colon cancer cell lines of human origin although quite frequent. To further investigate the phenomenon, two colon cancer cell lines were used, Caco-2/15 and HT-29, and share the trait of undergoing a differentiation program, characterized by a significant decline in proliferation, as a consequence of culture method. These two cell types express both β1 and β4 subunits but because the affinity of α6 for β4 is much greater than for β1, it can be assumed based on studies performed on keratinocytes and other cell types that the α6A/B variants mainly combine with β4 (Basora et al., 1999; Hogervorst et al., 1993; Hemler et al., 1989). This was confirmed by immunoprecipitating the α6B complex from metabolically labeled Caco-2/15 or primary keratinocytes with ^{[35S]} methionine and cystine using the α6 subunit-specific G0H3 antibody and analysis by SDS-PAGE under reduced and non-reduced conditions. As shown in Fig. 10B, Caco-2/15 cells display bands at approximately 205 kD and 150 kD and 205 kD and 120 kD corresponding to the β4 and α6 subunits under non-reduced and reduced conditions, respectively. As for the keratinocytes, the β1 subunit remained below the detection level in Caco-2/15 cells confirming that α6 predominantly associates with β4 in intestinal cells. Two intestinal cell models were used (Caco-2/15 and HT-29) to monitor the expression of the α6A and B variants during the course of proliferation arrest accompanied by the induction of differentiation. Competitive RT-PCR revealed that the ratio of α6A to α6B was altered as a function of cell state, reflecting the situation *in vivo.* Higher levels of α6A were associated with actively growing cells and a subsequent switch in favor of α6B was found to be associated with cells that had undergone cell cycle arrest (Fig. 10C). This trend was mirrored in protein levels as well when detected with variant specific antibodies (Fig. 10D, E).

*a6B inhibits proliferation in colon cancer cells.* The well-characterized colon cancer cell line Caco-2/15 which has the ability to constitutively deposit significant amounts of laminins (Vachon and Beaulieu, 1995) (the α6β4 ligand), was used to evaluate the hypothesis that an altered α6A/α6B ratio could be of functional importance for the proliferative status of colon cancer cells. The creation of a stable cell lines overexpressing α6A and α6B was attempted. However, the maintenance of the α6B cells in long term cultures was not possible, suggesting that overexpression of the α6B variant impaired cell proliferation. The nature of the mRNA splicing events underlying the formation of the two variants (exon exclusion leads to formation of the α6B variant) unfortunately precluded the specific depletion of the α6B variant by RNAi. Studies on the nucleofected cell populations were performed shortly following the 10-day antibiotic selection period ensuring that all cells expressed their respective episomal vectors (Fig. 11A). Proliferation was assessed using BrdU incorporation assays. It was observed that the proportion of α6B transfectants entering S-phase was significantly diminished compared to the α6A transfectants and the empty vector control (Fig. 11B), suggesting that predominant expression of the α6B subunit inhibits S-phase entry in intestinal epithelial cells in accordance with the predominant expression of this subunit in the non-proliferative compartment of the colon and it's relative down regulation in colon carcinomas. Consistent with these observations, a specific reduction in transcriptional Rb activity was observed in the luciferase assays (Fig. 12A) supporting the observation that the α6B-expressing cells are exiting "cell cycle in G1 (Harbour and Dean, 2000).

*α6B inhibits c-Myc activity in colon cancer cells.* Colon cancer cells and glandular proliferative cells require the activity of the proto-oncogene c-Myc for proliferation (van de Wetering et al., 2002). The ability of the α6B subunit to modulate c-Myc activity was then evaluated by performing co-transfections of the two integrin α6 variants with a luciferase reporter plasmid responding to c-Myc activity (pMyc-Ta-Luc). Experiments performed in colon cancer cells (Caco-2/15) demonstrated that pMyc-TA-luc activity was significantly down-regulated by α6B overexpression (Fig. 12B), but was not altered by α6A. The decrease in c-Myc activity in the α6B co-transfections was not due to any changes in c-Myc protein levels as determined by western blot analyses (Fig 12C).

The functional importance of the instructive interactions between the epithelial cell compartment with the surrounding extracellular milieu has long been recognized in the intestinal tract (Beaulieu, 1997; Teller and Beaulieu, 2001). Observations that the α6A and α6B splice variants are differentially expressed in progenitor and mature cells of the colonic epithelium, located in the lower and upper half of the glands respectively (Babyatsky and Podolsky, 1995), are consistent with previous findings showing the existence of distinct microenvironments between the proliferative and differentiation compartment in the gut (Chung and Mercurio, 2004; Basora et al., 1997). More importantly, it suggests that the intrinsic role of each α6 variant is distinct. The finding that the α6B variant exclusively repressed proliferation concurs with its low level of expression in the proliferative zone and its predominance in the non-proliferative compartments of the colon as shown herein, as well as in the small intestine (Dysenborg et al., 2009).

Colon cancer is the third leading cause of cancer related mortality in the United States (Jemal et al., 2008). The occurrence and development of most colon cancers follows a stereotypical pattern of a progressive accumulation of gainand loss of function mutations of oncogenes and tumor suppressor genes (Radke and Clevers, 2005). It is also clear that neoplastic cells tend to up-regulate the expression of integrins favoring their migration, survival and proliferation during the complex multistep generation of tumors (Guo and Giancotti, 2004). The mechanistic and biochemical roles of the α6β4 integrin in carcinoma biology are well documented (Mercurio and Rabinovitz, 2001) and the up-regulation in primary colon carcinomas of the β4 (Ni et al., 2005) and, as shown here, the α6 subunit reflects the importance of this integrin in colon cancer progression. The results presented herein describe novel aspects of the biology of the α6β4 integrin variants in colon cancer. Thus, after having identified that distinct forms of the integrin β4 subunit were expressed in normal intestinal proliferative vs cancer cells (Ni et al., 2005), it was observed that there is a shift in α6 variant expression from a predominantly high α6B/α6A ratio in the normal colon to a predominantly low α6B/α6A ratio in primary tumors.

c-Myc is a key player in cancer formation and progression due to the numerous roles it plays in proliferation control and apoptosis (Adhikary and Eilers, 2005). Indeed, c-Myc expression has been demonstrated to be upregulated in 70% of colon cancers (Erisman et al., 1985) and its well documented effects include stimulation of cyclinD expression, inhibition of p21CIP1 and p27KIP1 expression and inactivation of Rb, leading to enhanced G1 to S-phase transition (Adhikary and Eilers, 2005). Given the central role of c-Myc in the control of critical cellular events, its transcriptional functions are tightly regulated by several molecular pathways (Cole and Nikiforov, 2006). Prototypically, the control of cellular c-Myc levels are largely attributed to the canonical activity of the Wnt-pathway (Clevers, 2006). However, the activity and expression levels of c-Myc are regulated by several other molecular mechanisms including distinct signaling pathways and interaction with binding partners (Adhikary and Eilers, 2005, Sakamuro and Prendergast, 1999). For instance, the nucleoshuttling scaffold protein Bridging Integrator-1 (Bin1) has been shown to strongly inhibit c-Myc transcriptional activity in a Wnt-pathway independent manner (Elliot et al, 1999; Kinney et al., 2008). Interestingly, Bin1 can selectively interact with the cytoplasmic domain of the α6B integrin subunit in yeast two-hybrid studies (Wixler et al., 1999) and its expression has been reported to be associated with the non-proliferative cell population in the normal intestine (DuHadaway et al., 2003) suggesting a possible mechanism for the inhibitory effect of α6Bβ4 on c-Myc activity.

While there is substantial evidence for the differential capacity of the α6Aβ1 and α6Bβ1 integrins to initiate intracellular signalling (Shaw et al., 1995; Wei et al., 1998) and facilitate migration on laminin (Shaw et al., 1995), these studies have all found that the α6Aβ1 integrin functions as the "active" integrin, whereas the α6Bβ1 integrin appears to have no major active role in these events (Shaw et al., 1995; Wei et al., 1998; Ferletta et al., 2003; Shaw et al., 1995; Guimond et al, 1998). In this context it is noteworthy that overexpression of the α6A variant in colon cancer cells did not stimulate proliferation as compared to the control cells, but rather the α6B variant actively inhibited proliferation and c-Myc activity. It is furthermore noteworthy that the α6A/B splice variants can dimerize with both the β1 and the β4 subunits to form two distinct functional integrins, α6β1 and α6β4, but that the α6 subunit preferentially dimerizes with the β4 subunit (Basora et al., 1999; Hogervost et al., 1993; Hemler et al., 1989), as confirmed herein. Thus, the specific ability of the α6B subunit to inhibit proliferation, in accordance with its predominant expression in the quiescent compartment of the normal colon and its down regulation in primary colon cancers and adenocarcinoma cell lines, strongly suggests that the expression and ratio of the α6A and α6B splice variants are inherent to normal intestinal homeostasis and exploited by colon cancer cells.

### EXAMPLE III - DOWNREGULATION OF THE α6A ISOFORM LIMITS IN VIVO TUMOR GROWTH

Caco-2/15 cells have been infected with a lentiviral-based system (MISSION™ from Sigma) containing the shRNA sequence GATCATTATGATGCCACATATC (SEQ ID NO: 13) for silencing exon A of the α6A isoform (Shα6A cells) or a control lentivirus (Shc cells). The TCF/β-catenin activity of these cells was then assessed using the TOP/FOP flash luciferase assay as described in Example II. Proliferation of these cells was also quantified with the BrdU assay, as described in Example II. In addition, these cells have been injected into immuno-compromised mice and the size of the tumor they generate has been measured as a function of time.

As shown in Figure 13A, the mRNA expression of the α6A isoform is abolished in Shα6A cells when compared to the Shc cells. Further, the lack of expression of the α6A isoform reduces the TCF/b-catenin activity as well as their proliferation rate (Fig. 13B and C, respectively). In addition, the reduced expression of the α6A isoform in the Shα6A cells reduces the size of the tumor as well as the time of appearance of those tumors (Fig. 13D).

Silencing exon A of the alpha6 isoform in four other colon cancer cell lines (HT-29, T84, SW480 and SW620) using same lentiviral infection procedure resulted in significant reduction of proliferation in three of the four cell lines as quantified with the BrdU assay (data not shown).

### REFERENCES

Adhikary S, Eilers M (2005) Transcriptional regulation and transformation by Myc proteins. Nat Rev Mol Cell Biol, 6:635-645.
Astler VB, Coller FA (1954) The prognostic significance of direct extension of carcinoma of the colon and rectum. Ann Surg, 139:846-851.
Babyatsky MW, Podolsky DK (1995) Growth and Development of the Gastrointestinal Tract. In: Yamada T (ed) Textbook of Gastroenterology. JB Lippincott, Philadelphia, pp 546-577.
Basora N, Herring-Gillam FE, Boudreau F, Perreault N, Pageot LP, Simoneau M, Bouatrouss Y, Beaulieu JF (1999) Expression of functionally distinct variants of the beta(4)A integrin subunit in relation to the differentiation state in human intestinal cells. J Biol Chem 274:29819-29825.
Basora N, Vachon PH, Herring-Gillam FE, Perreault N, Beaulieu JF (1997) Relation between integrin alpha7Bbeta1 expression in human intestinal cells and enterocytic differentiation. Gastroenterology 113:1510-1521.
Beaulieu JF, Quaroni A (1991) Clonal analysis of sucrase-isomaltase expression in the human colon adenocarcinoma Caco-2 cells. Biochem J, 280 (Pt 3):599-608.
Beaulieu JF (1992) Differential expression of the VLA family of integrins along the crypt-villus axis in the human small intestine. J Cell Sci, 102 (Pt 3):427-436.
Beaulieu JF (1997) Extracellular matrix components and integrins in relationship to human intestinal epithelial cell differentiation. Prog Histochem Cytochem 31:1-78.
Boudreau F, Rings EH, van Wering HM, Kim RK, Swain GP, Krasinski SD, Moffett J, Grand RJ, Suh ER, Traber PG (2002) Hepatocyte nuclear factor-1 alpha, GATA-4, and caudal related homeodomain protein Cdx2 interact functionally to modulate intestinal gene transcription. Implication for the developmental regulation of the sucrase-isomaltase gene. J Biol Chem 277:31909-31917.
Clevers H (2006) Wnt/beta-catenin signaling in development and disease. Cell, 127:469-480.
Cole MD, Nikiforov MA (2006) Transcriptional activation by the Myc oncoprotein. Curr Top Microbiol Immunol, 302:33-50.
Chung J, Mercurio AM (2004) Contributions of the alpha6 integrins to breast carcinoma survival and progression. Mol Cells, 17:203-209.
de Curtis I, Reichardt LF (1993) Function and spatial distribution in developing chick retina of the laminin receptor alpha 6 beta 1 and its isoforms. Development 118:377-388.
de Melker AA, Sonnenberg A (1999) Integrins: alternative splicing as a mechanism to regulate ligand binding and integrin signaling events. Bioessays 21:499-509.
Delwel GO, Hogervorst F, Kuikman I, Paulsson M, Timpl R, Sonnenberg A (1993) Expression and function of the cytoplasmic variants of the integrin alpha 6 subunit in transfected K562 cells. Activation-dependent adhesion and interaction with isoforms of laminin. J Biol Chem 268:25865-25875.
DuHadaway JB, Lynch FJ, Brisbay S, Bueso-Ramos C, Troncoso P, McDonnell T, Prendergast GC (2003) Immunohistochemical analysis of Bin1/Amphiphysin II in human tissues: diverse sites of nuclear expression and losses in prostate cancer. J Cell Biochem, 88:635-642.
Dydensborg AB, Herring E, Auclair J, Tremblay E, Beaulieu JF (2006) Normalizing genes for quantitative RT-PCR in differentiating human intestinal epithelial cells and adenocarcinomas of the colon. Am J Physiol Gastrointest Liver Physiol, 290:G1067-1074.
Dydensborg AB, Teller IC, Basora N, Groulx JF, Auclair J, Francoeur C, Escaffit F, Paré F, Herring E, Ménard D, Beaulieu JF (2009) Differential expression of the integrin alpha6Abeta4 and alpha6Bbeta4 along the crypt-villus axis in the human small intestine. Histochem Cell Biol , 131:531-536.
Elliott K, Sakamuro D, Basu A, Du W, Wunner W, Staller P, Gaubatz S, Zhang H, Prochownik E, Eilers M, Prendergast GC (1999) Bin1 functionally interacts with Myc and inhibits cell proliferation via multiple mechanisms. Oncogene, 18:3564-3573.
Erisman MD, Rothberg PG, Diehl RE, Morse CC, Spandorfer JM, Astrin SM (1985) Deregulation of c-myc gene expression in human colon carcinoma is not accompanied by amplification or rearrangement of the gene. Mol Cell Biol, 5:1969-1976.
Escaffit F, Boudreau F, Beaulieu JF (2005) Differential expression of claudin-2 along the human intestine: Implication of GATA-4 in the maintenance of claudin-2 in differentiating cells. J Cell Physiol 203:15-26.
Ferletta M, Kikkawa Y, Yu H, Talts JF, Durbeej M, Sonnenberg A, Timpl R, Campbell KP, Ekblom P, Genersch E (2003) Opposing roles of integrin alpha6Abeta1 and dystroglycan in laminin-mediated extracellular signal-regulated kinase activation. Mol Biol Cell, 14:2088-2103.
Friedrichs K, Ruiz P, Franke F, Gille I, Terpe HJ, Imhof BA (1995) High expression level of alpha 6 integrin in human breast carcinoma is correlated with reduced survival. Cancer Res, 55:901-906.
Giancotti FG, Tarone G (2003) Positional control of cell fate through joint integrin/receptor protein kinase signaling. Annu Rev Cell Dev Biol 19:173-206.
Gimond C, Baudoin C, van der Neut R, Kramer D, Calafat J, Sonnenberg A (1998) Cre-loxP-mediated inactivation of the alpha6A integrin splice variant in vivo: evidence for a specific functional role of alpha6A in lymphocyte migration but not in heart development. J Cell Biol, 143:253-266.
Guo W, Giancotti FG (2004) Integrin signalling during tumour progression. Nat Rev Mol Cell Biol, 5:816-826.
Harbour JW, Dean DC (2000) Rb function in cell-cycle regulation and apoptosis. Nat Cell Bioi, 2:E65-67.
Hemler ME, Crouse C, Sonnenberg A (1989) Association of the VLA alpha 6 subunit with a novel protein. A possible alternative to the common VLA beta 1 subunit on certain cell lines. J Bio) Chem, 264:6529-6535. °
Hogervorst F, Admiraal LG, Niessen C, Kuikman I, Janssen H, Daams H, Sonnenberg A (1993) Biochemical characterization and tissue distribution of the A and B variants of the integrin alpha 6 subunit. J Cell Bbl 121:179-191.
Hogervorst F, Kuikman I, van Kessel AG, Sonnenberg A (1991) Molecular cloning of the human alpha 6 integrin subunit. Alternative splicing of alpha 6 mRNA and chromosomal localization of the alpha 6 and beta 4 genes. Eur J Biochem 199:425-433.
Ilyas M, Tomlinson IP, Rowan A, Pignatelli M, Bodmer Wf (1997) Beta-catenin mutations in cell lines established from human colorectal cancers. Proc Natl Acad Sci U S A 94:10330-10334.
Jemal A, Siegel R, Ward E, Hao Y, Xu J, Murray T, Thun MJ (2008) Cancer statistics, 2008. CA Cancer J Clin, z58:71-96.
Kinney EL, Tanida S, Rodrigue AA, Johnson JK, Tompkins VS, Sakamufo D (2008) Adenovirus E1A oncoprotein liberates c-Myc activity to promote cell proliferation through abating Bin1 expression via an Rb/E2F1-dependent mechanism. J Gell Physiol, 216:Ni21-631.
Korinek V, Barker N, Morin PJ, van Wichen D, de Weger R, Kinzler KW, Vogelstein B, Clevers H (1997) Constitutive transcriptional activation by a beta-catenin-Tcf complex in APC-/- colon carcinoma. Science 275:1784-1787.
Lohi J, Oivula J, Kivilaakso E, Kiviluoto T, Frojdman K, Yamada Y, Burgeson RE, Leivo I, Virtanen I (2000) Basement membrane laminin-5 is deposited in colorectal adenomas and carcinomas and serves as a ligand for alpha3beta1 integrin. Apmis 108:161-172.
Lorenzen JA, Bonacci BB, Palmer RE, Wells C, Zhang J, Haber DA, Goldstein AM, Mayer AN (2005) Rbm19 is a nucleolar protein expressed in crypt/progenitor cells of the intestinal epithelium. Gene Expr Patterns, 6:45-56.
Mercurio AM, Bachelder RE, Rabinovitz I, O'Connor KL, Tani T, Shaw LM (2001) The metastatic odyssey: the integrin connection. Surg Oncol Clin N Am, 10:313-328, viii-ix.
Mercurio AM, Rabinovitz I (2001) Towards a mechanistic understanding of tumor invasion--lessons from the alpha6beta 4 integrin. Semin Cancer Biol 11:129-141.
Ni H, Dydensborg AB, Herring FE, Basora N, Gagne D, Vachon PH, Beaulieu JF (2005) Upregulation of a functional form of the beta4 integrin subunit in colorectal cancers correlates with c-Myc expression. Oncogene 24:6820-6829.
Olsen L, Bressendorff S, Troelsen JT, Olsen J (2005) Differentiation-dependent activation of the human intestinal alkaline phosphatase promoter by HNF-4 in intestinal cells. Am J Physiol Gastrointest Liver Physiol 289:G220-226.
Perreault N, Beaulieu J (1996) Use of the dissociating enzyme thermolysine to generate viable human normal intestinal epithelial cell cultures. Exp Cell Res 224:354-364.
Perreault N, Beaulieu JF (1998) Primary cultures of fully differentiated and pure human intestinal epithelial cells. Exp Cell Res 245:34-42.
Pouliot N, Nice EC, Burgess AW (2001) Laminin-10 mediates basal and EGF-stimulated motility of human colon carcinoma cells via alpha(3)beta(1) and alpha(6)beta(4) integrins. Exp Cell Res 266:1-10.
Radtke F, Clevers H: Self-renewal and cancer of the gut: two sides of a coin. Science 2005, 307:1904-1909.
Sakamuro D, Prendergast GC: New Myc-interacting proteins: a second Myc network emerges. Oncogene 1999, 18:2942-2954.
Segat D, Comai R, Di Marco E, Strangio A, Cancedda R, Franzi AT, Tacchetti C (2002) Integrins alpha 6Abeta 1 and alpha 6Bbeta 1 Promote Different Stages of Chondrogenic Cell Differentiation. J Biol Chem 277:31612-31622.
Shaw LM, Mercurio AM (1995) Regulation of alpha 6 beta 1 integrin-mediated migration in macrophages. Agents Actions Suppl 47:101-106.
Shaw LM, Rabinovitz I, Wang HH, Toker A, Mercurio AM (1997) Activation of phosphoinositide 3-OH kinase by the alpha6beta4 integrin promotes carcinoma invasion. Cell, 91:949-960.
Shaw LM, Turner CE, Mercurio AM (1995) The alpha 6A beta 1 and alpha 6B beta 1 integrin variants signal differences in the tyrosine phosphorylation of paxillin and other proteins. J Biol Chem 270:23648-23652.
Sonnenberg A, Janssen H, Hogervorst F, Calafat J, Hilgers J (1987) A complex of platelet glycoproteins Ic and Ila identified by a rat monoclonal antibody. J Biol Chem 262:10376-10383.
Teller IC, Beaulieu JF (2001) Interactions between laminin and epithelial cells in intestinal health and disease. Expert Rev Mol Med 2001:1-18.
Thorsteinsdottir S, Roelen BA, Freund E, Gaspar AC, Sonnenberg A, Mummery CL (1995) Expression patterns of laminin receptor splice variants alpha 6A beta 1 and alpha 6B beta 1 suggest different roles in mouse development. Dev Dyn 204:240-258.
Troelsen JT, Olsen J, Moller J, Sjostrom H (2003) An upstream polymorphism associated with lactase persistence has increased enhancer activity. Gastroenterology 125:1686-1694.
Vachon PH, Beaulieu JF (1995) Extracellular heterotrimeric laminin promotes differentiation in human enterocytes. Am J Physiol 268:G857-867.
Vandesompele J, De Preter K, Pattyn F, Poppe B, Van Roy N, De Paepe A, Speleman F: Accurate normalization of real-time quantitative RT-PCR data by geometric averaging of multiple internal control genes. Genome Biol 2002, 3:RESEARCH0034.
van de Wetering M, Sancho E, Verweij C, de Lau W, Oving I, Hurlstone A, van der Horn K, Batlle E, Coudreuse D, Haramis AP (2002) The beta-catenin/TCF-4 complex imposes a crypt progenitor phenotype on colorectal cancer cells. Cell, 111:241-250.
Virtanen I, Gullberg D, Rissanen J, Kivilaakso E, Kiviluoto T, Laitinen LA, Lehto VP, Ekblom P (2000) Laminin alpha1-chain shows a restricted distribution in epithelial basement membranes of fetal and adult human tissues. Exp Cell Res 257:298-309.
Wei J, Shaw LM, Mercurio AM (1998) Regulation of mitogen-activated protein kinase activation by the cytoplasmic domain of the alpha6 integrin subunit. J Biol Chem 273:5903-5907.
Wixler V, Laplantine E, Geerts D, Sonnenberg A, Petersohn D, Eckes B, Paulsson M, Aumailley M (1999) Identification of novel interaction partners for the conserved membrane proximal region of alpha-integrin cytoplasmic domains. FEBS Lett, 445:351-355.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth.

## Claims

1. An ex vivo method of diagnosing a colon cancer in an individual, said method comprising determining a ratio between an isoform B and an isoform A of an integrin α6 subunit in a cell from said individual, wherein the colon cancer in said individual is diagnosed if the ratio is lower than a control ratio.

2. The method of claim 1, wherein the ratio between the isoform B and the isoform A of the integrin α6 subunit is determined by quantifying the mRNA specific for the isoform B and the mRNA specific for the isoform A in said cell or by quantifying the polypeptide specific for the isoform B and the polypeptide specific for the isoform A in said cell.

3. The method of claim 1, wherein the control ratio is a ratio between the isoform B and the isoform A of the integrin α6 subunit in a cell from a healthy control patient or a healthy tissue free of cancer.

4. The method of claim 1, wherein the colon cancer is a carcinoma.

## Patentansprüche

1. Ex-vivo-Verfahren zur Diagnose eines Kolonkrebses in einem Individuum, wobei das Verfahren Folgendes umfasst: Bestimmen eines Verhältnisses zwischen einer Isoform B und einer Isoform A einer Integrin-α6-Untereinheit in einer Zelle des Individuums, wobei der Kolonkrebs in dem Individuum dann diagnostiziert wird, wenn das Verhältnis unter einem Kontrollverhält-nis liegt.

2. Verfahren nach Anspruch 1, wobei das Verhältnis zwischen der Isoform B und der Isoform A der Integrin-α6-Untereinheit bestimmt wird, indem die mRNA, die spezifisch für die Isoform B ist, und die mRNA, die spezifisch für die Isoform A ist, in der Zelle quantifiziert werden, oder indem das Polypeptid, das spezifisch für die Isoform B ist, und das Polypeptid, das spezifisch für die Isoform A ist, in der Zelle quantifiziert werden.

3. Verfahren nach Anspruch 1, wobei das Kontrollverhältnis ein Verhältnis zwischen der Isoform B und der Isoform A der Integrin-α6-Untereinheit in einer Zelle von einem gesunden Kontroll-patienten oder einem gesunden, krebsfreien Gewebe ist.

4. Verfahren nach Anspruch 1, wobei der Kolonkrebs ein Karzinom ist.

## Revendications

1. Procédé de diagnostic *ex vivo* d'un cancer du côlon chez un individu, ledit procédé comprenant la détermination d'un ratio entre une isoforme B et une isoforme A d'une sous-unité d'intégrine α6 dans une cellule provenant dudit individu, le cancer du côlon chez ledit individu étant diagnostiqué si le ratio est inférieur à un ratio de contrôle.

2. Procédé selon la revendication 1, dans lequel le ratio entre l'isoforme B et l'isoforme A de la sous-unité d'intégrine α6 est déterminé par quantification de l'ARNm propre à l'isoforme B et de l'ARNm propre à l'isoforme A dans ladite cellule, ou par quantification du polypeptide propre à l'isoforme B et du polypeptide propre à l'isoforme A dans ladite cellule.

3. Procédé selon la revendication 1, dans lequel le ratio de contrôle est un ratio entre l'isoforme A et l'isoforme B de la sous-unité d'intégrine α6 dans une cellule provenant d'un patient témoin sain ou d'un tissu sain exempt de cancer.

4. Procédé selon la revendication 1, dans lequel le cancer du côlon est un carcinome.
